# EUROPEAN PATENT APPLICATION

(11) **EP 4 803 510 A1**
(43) Date of publication of application: **09.09.2026**
(21) Application number: 25162275.9
(22) Date of filing: 07.03.2025
(51) Int. Cl.: C07D 311/22, A61K 31/353, A61P 35/00, A61P 35/02

(54) **SYNTHETIC DERIVATIVES OF NATURAL PRODUCTS WITH ANTICANCER ACTIVITY**

(71) Applicant: Floratek Pharma SA, 1170 Aubonne (CH)
(72) Inventor: STOICESCU, Dan Florin, 1170 Aubonne (CH)
(74) Representative: Braunpat AG

(57) **Abstract**

The present invention relates to flavonoid derivatives of the general formula (I):

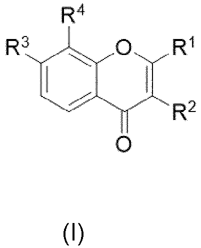

in a free form, or in a pharmaceutically acceptable salt form. The compounds of general formula (I) are useful in medicine, in particular for the prevention and treatment of cancer.

## Description

### FIELD OF THE INVENTION

The present invention relates to synthetic flavonoid derivatives and pharmaceutical compositions containing these compounds. It focuses on their therapeutic uses, especially for the prevention and treatment of cancer.

### BACKGROUND OF THE INVENTION

Flavonoids are polyphenolic compounds produced in plants as bioactive secondary metabolites. They contribute to the plant's color and flavor and also have significant pharmacological effects. Flavonoids exhibit a range of anticancer properties, including:
- Modulating reactive oxygen species (ROS)-scavenging enzyme activity
- Inducing cell cycle arrest
- Inducing apoptosis
- Inducing autophagy
- Suppressing cancer cell proliferation, and
- Suppressing cancer cell invasiveness.

However, natural flavonoids have significant limitations in clinical use. They often show low bioavailability and insufficient specificity for cancer cells. Moreover, their broad biological activity leads to complex mechanisms of action. These mechanisms depend on a delicate balance between pro-oxidant and antioxidant effects, as well as other effects that can be both beneficial and adverse.

To overcome these limitations, patent applications WO2022/167698A1 and WO2022/167696A1 introduced flavonoid derivatives that incorporate a polyamine chain from the spermidine class. These compounds demonstrated over 50% inhibitory activity at a 2 µM concentration against a broad range of cancer cell lines, including those derived from brain, colon, leukemia, lung, lymphoma, muscle, and melanoma.

Despite these advances, there remains a need for novel anticancer agents with enhanced potency and broader-spectrum efficacy. In particular, such agents should be effective against cancer types that are resistant to current therapies (e.g., chemotherapy, targeted therapy, hormone therapy, or radiation therapy). They should also exhibit improved pharmacokinetic properties.

### SUMMARY OF THE INVENTION

Accordingly, the present invention provides a flavonoid compound of general formula (I): 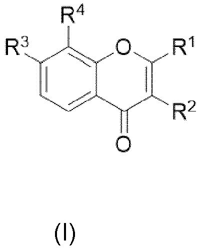 wherein
either R¹ represents A, and R² is selected from -H, -OCH₃, -OCH₂F, -OCHF₂, and -OCF₃, or
R² represents A, and R¹ is selected from -H, -OCH₃, -OCH₂F, -OCHF₂, and -OCF₃,
A represents 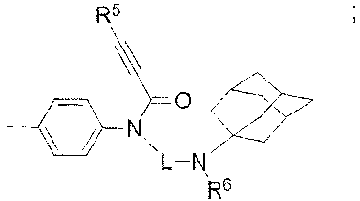
R₃ and R₄ are independently of each other selected from -O(C₁-C₆alkyl), a halogenated -O(C₁-C₆alkyl), a halogen atom and -NR⁷R⁸;
R⁵ is selected from -H, -C₁-C₆alkyl and a silyl group;
-L- represents -(CH₂)n- with n being an integer from 2 to 10;
R⁶ is selected from -C₁-C₄ alkyl;
R⁷ and R⁸ are independently selected from -C₁-C₆ alkyl; and the compound is in a free form, or in a pharmaceutically acceptable salt form.

A second aspect according to the present invention is directed to a pharmaceutical composition containing the compound of general formula (I) and one or more excipients.

A third aspect according to the present invention is directed to a compound of general formula (I), or a pharmaceutical composition containing the compound of general formula (I) and one or more excipients for use as a medicament.

A fourth aspect according to the present invention is directed to a compound of general formula (I), or a pharmaceutical composition containing the compound of general formula (I) and one or more excipients for use for use in the treatment or prevention of cancer, including therapy-resistant (e.g., chemotherapy-resistant, targeted-therapy resistant, hormone-therapy resistant, radiation-therapy resistant) cancer forms and rare cancer forms.

### DETAILED DESCRIPTION OF THE INVENTION

This invention aims to meet the need for novel anticancer agents that demonstrate enhanced efficacy against a broad range of cancer types-particularly therapy-resistant forms (e.g., chemotherapy-, targeted-therapy-, hormone-therapy-, and radiation-therapy-resistant) and rare cancer types, while exhibiting favorable pharmacokinetic properties. This objective is accomplished by the compound described in claim 1 and the pharmaceutical composition described in claim 12. Preferred embodiments are outlined in the specification and further detailed in the dependent claims.

The present invention will be described in more detail below.

Where the present description refers to "preferred" embodiments/features, combinations of these "preferred" embodiments/features are also deemed to be disclosed as long as the specific combination of the "preferred" embodiments/features is technically meaningful.

Unless otherwise stated, the following definitions shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the term "and/or" means that either all or only one of the elements of said group may be present. For example, "A and/or B" means "only A, or only B, or both A and B". In the case of "only A", the term also covers the possibility that B is absent, i.e. "only A, but not B".

As used herein, the terms "including", "containing" and "comprising" are used herein in their open-ended, non-limiting sense. It is understood that the various embodiments, preferences and ranges may be combined at will. Thus, for instance a solution comprising a compound A may include other compounds besides A. However, the term "comprising" also covers, as a particular embodiment thereof, the more restrictive meanings of "consisting essentially of" and "consisting of, so that for instance "a solution comprising A, B and optionally C" may also (essentially) consist of A and B, or (essentially) consist of A, B and C. As used herein, the transitional phrase "consisting essentially of" (and grammatical variants) is to be interpreted as encompassing the recited materials or steps and those that do not materially affect the basic and novel characteristic(s) of the claimed invention. Thus, the term "consisting essentially of" should not be interpreted as equivalent of "comprising".

It has been found that a compound of general formula (I): 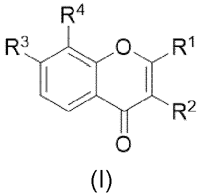 wherein
either R¹ represents A, and R² is selected from -H, -OCH₃, -OCH₂F, -OCHF₂, and -OCF₃, or
R² represents A, and R¹ is selected from -H, -OCH₃, -OCH₂F, -OCHF₂, and -OCF₃,
A represents 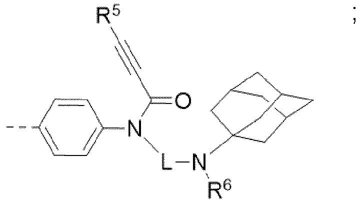
R₃ and R₄ are independently of each other selected from -O(C₁-C₆alkyl), a halogenated -O(C₁-C₆alkyl), a halogen atom and -NR⁷R⁸;
R⁵ is selected from -H, -C₁-C₆alkyl and a silyl group;
-L- represents -(CH₂)n- with n being an integer from 2 to 10;
R⁶ is selected from -C₁-C₄alkyl;
R⁷ and R⁸ are independently selected from -C₁-C₆alkyl; and the compound is in a free form, or in a pharmaceutically acceptable salt form, exhibits a broad-spectrum anticancer efficacy, including efficacy against therapy-resistant cancer forms (e.g., cancer forms with mutated binding sites or altered drug efflux mechanisms) and rare cancer forms, and shows significantly lower *in vitro* IC₅₀ values than known anticancer agents, and improved pharmacokinetic properties.

It is a reasonable assumption that the high potency of the compound of formula (I) is due to the covalent binding between the alkyne residue present in the propiolamide functional group and the thiol group of the cysteine residues present in oncogenic and survival-related proteins. The propiolamide functional group allows for highly specific and irreversible interaction with cysteine-containing oncogenic and survival-related protein targets, ensuring long-lasting inhibition and *in vitro* picomolar-range IC₅₀ values. As drugs that form covalent bonds with proteins latch irreversibility onto their targets, they ensure a longer action time and are effective in smaller doses than noncovalent drugs.

Advantageously, the flavonoid backbone enhances metabolic stability and bioavailability, providing improved pharmacokinetic properties over known anticancer covalent inhibitors, such as Ibrutinib, an irreversible Bruton's tyrosine kinase (BTK) inhibitor for hematologic cancers, Osimertinib, a covalent epidermal growth factor receptor (EGFR) inhibitor effective against resistant non-small cell lung cancer (NSCLC), and Sotorasib, a covalent KRAS G12C inhibitor.

Moreover, unlike Ibrutinib and Isimertinib, which lose efficacy in resistant cancer populations, the compound described herein maintains its inhibitory activity in cancer cells that have developed resistance to standard covalent inhibitors, including forms with mutated binding sites or altered drug efflux mechanisms.

As used herein, the terms "salt" or "salts" refers to an acid addition or base addition salt of the compound of general formula (I). "Salts" include in particular "pharmaceutically acceptable salts". The term "pharmaceutically acceptable salts" refers to salts that retain the biological effectiveness and properties of the compound of this invention and, which typically are not biologically or otherwise undesirable. The compound of formula (I) of the present invention is capable of forming a characteristic salt with a defined acid by virtue of the presence of a basic tertiary amine in its structure.

Pharmaceutically acceptable acid addition salts may be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, glycolic, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

In a preferred embodiment, the compound of general formula (I) is in a pharmaceutically acceptable salt form, preferably selected from a hydrochloride salt form, a hydrobromide form, a mesylate salt form, an acetate salt form, and a trifluoroacetate salt form.

A preferred compound according to the present invention is a flavonoid compound of general formula (I-A) 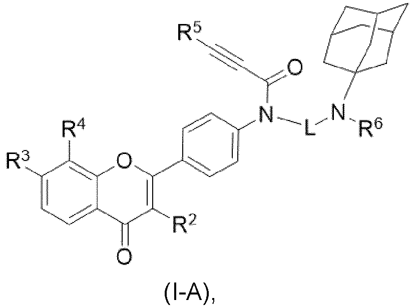 wherein R², R³, R⁴, R⁵, R⁶ and L have the meanings as defined herein.

A further preferred compound according to the present invention is a compound of general formula (I-B) 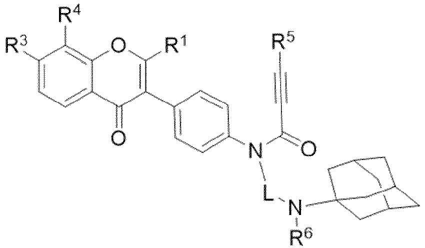 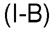 wherein R¹, R³, R⁴, R⁵, R⁶ and L have the meanings as defined herein.

In one preferred embodiment according to the present invention R⁵ represents -H.

In a further preferred embodiment according to the present invention, R⁵ is a silyl group, preferably a silyl group of formula -SiR⁹R¹⁰R¹¹ with R⁹, R¹⁰ and R¹¹ being independently selected from -C₁-C₆alkyl and phenyl, preferably selected from methyl, ethyl, iso-propyl, iso-butyl, tert-butyl, thexyl and phenyl. Thus, a preferred embodiment according to the present invention is directed to a compound of general formula (I), (I-A) or (I-B), wherein R⁵ represents a silyl group, preferably of general formula -SiR⁹R¹⁰R¹¹, with R⁹, R¹⁰ and R¹¹ having the meanings described herein. The silyl group is preferably selected from trimethylsilyl (TMS), triethylsilyl (TES), tert-butyldimethylsilyl (TBS), thexyldimethylsilyl (TDS), tert-butyldiphenylsilyl (TBDPS), triisopropylsilyl (TIPS) and di-*tert*-butylisobutylsilyl (BIBS), more preferably from trimethylsilyl (TMS), triethylsilyl (TES), tert-butyldimethylsilyl (TBS), and triisopropylsilyl (TIPS), much preferably from trimethylsilyl (TMS), tertbutyldimethylsilyl (TBS) and triisopropylsilyl (TIPS), in particular trimethylsilyl (TMS), and tertbutyldimethylsilyl (TBS).

In the compounds described herein, R₃ and R₄ are independently of each other selected from -O(C₁-C₆alkyl), a halogenated -O(C₁-C₆alkyl), a halogen atom (e.g., F, Cl, Br, I) and -NR⁷R⁸, with R⁷ and R⁸ being independently selected from -C₁-C₆alkyl, preferably from -O(C₁-C₃alkyl), a halogenated -O(C₁-C₃alkyl), a halogen atom, in particular F and Cl, and -NR⁷R⁸, with R⁷ and R⁸ being independently selected from -C₁-C₃alkyl.

As well known to the skilled person, a -C₁-C₆alkyl substituent group or moiety in a substituent refers to an alkyl group/moiety having from 1 to 6 carbon atoms. The alkyl group/moiety may be linear or branched. Examples of a -C₁-C₆alkyl group/moiety include methyl, ethyl, n-propyl, iso-propyl, n-butyl, iso-butyl, tert-butyl, pentyl and hexyl groups/moieties.

Examples of -O(C₁-C₆alkyl) group/moiety include, but are not limited to, -OCH₃, -OCH₂CH₃, -OCH₂CH₂CH₃, and -OCH(CH₃)(CH₃).

As used herein, a halogenated -O(C₁-C₆alkyl) group or moiety in a substituent refers to an -O(C₁-C₆alkyl) group/moiety wherein one or more hydrogen atoms in the -C₁-C₆alkyl group/moiety have been replaced by a halogen atom, e.g., F, Cl, Br or I. Examples include, but are not limited to: -OCH₂F, -OCHF₂, -OCF₃, -OCH₂Cl, -OCHCl₂, -OCCl₃, -OCH₂CH₂F, -OCH₂CH₂CH₂F, and -OCH(CH₃)(CH₂F).

In a preferred embodiment, R₃ and R₄ are independently of each other selected from -OCH₃, -OCH₂F, -OCHF₂, -OCF₃, -F, -Cl and -NR⁷R⁸, with R⁷ and R⁸ being preferably methyl -CH₃.

A compound of general formula (I), (I-A) or (I-B), wherein
- R³ and R⁴ represent -OCH₃, -OCH₂F, -OCHF₂, or -OCF₃, preferably -OCH₃; or
- R³ represents -N(CH₃)₂ and R⁴ represents -OCH₃, -OCH₂F, -OCHF₂, or -OCF₃, preferably -OCH₃; or
- R³ represents F and R⁴ represents CI; or
- R³ represents -OCH₃, -OCH₂F, -OCHF₂, or -OCF₃, preferably -OCH₃, and -N(CH₃)₂ and R⁴ represents -N(CH₃)₂;
are preferred. Advantageously, a compound of general formula (I), (I-A) or (I-B), wherein
- R³ represents -N(CH₃)₂ and R⁴ represents -OCH₃, -OCH₂F, -OCHF₂, or -OCF₃, preferably -OCH₃; or
- R³ represents F and R⁴ represents Cl; exhibits improved pharmacokinetic properties.

In the compounds described herein R⁶ is selected from -C₁-C₄alkyl, preferably from -C₁-C₃alkyl, more preferably from -CH₃ and -CH₂CH₃, much preferably is -CH₃.

In the compounds described herein, -L- represents -(CH₂)n- with n being an integer from 2 to 10. n may be an integer from 3 to 9, from 3 to 8, from 3 to 7, from 3 to 6, such as 4.

Preferably the compound of general formula (I) is selected from:

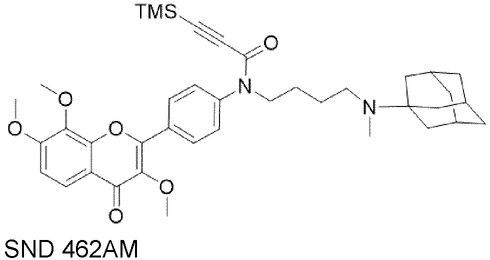

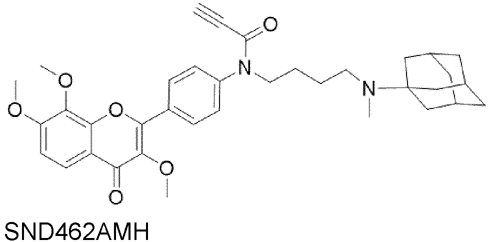

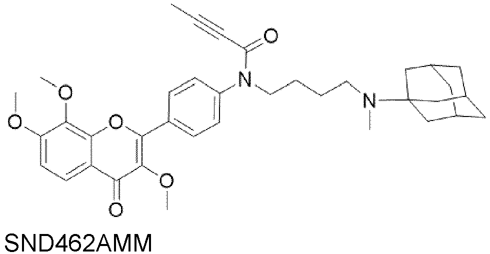

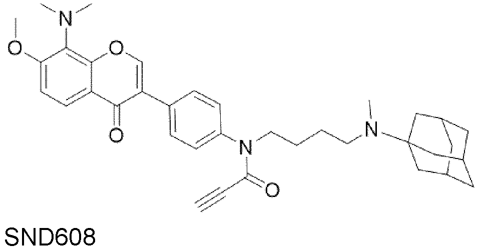

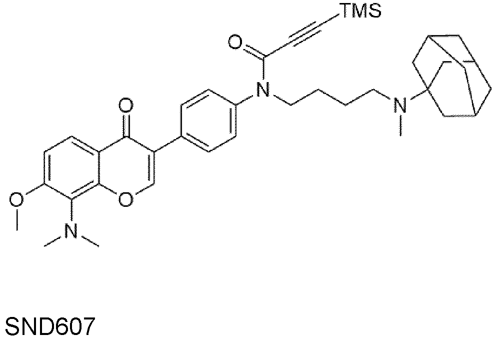

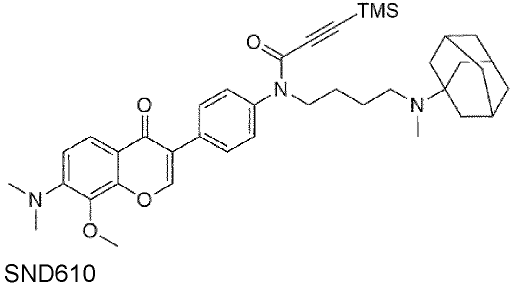

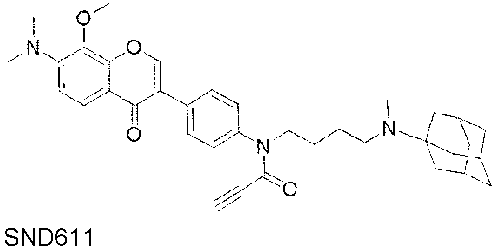

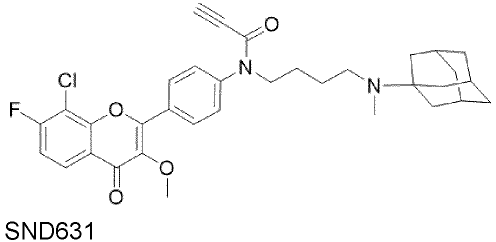

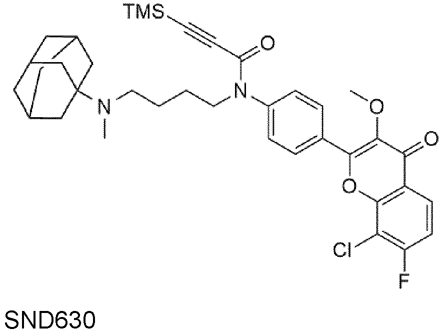

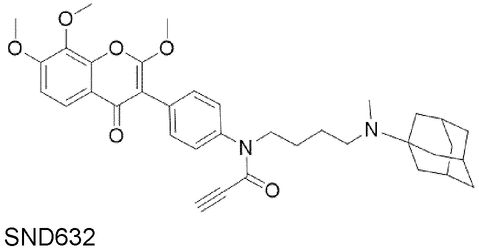

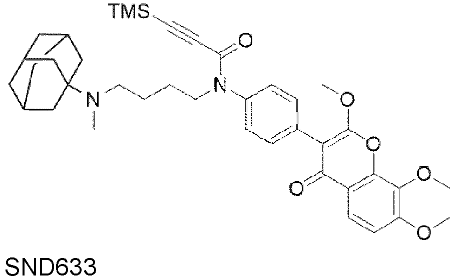

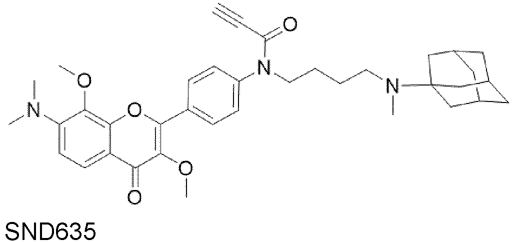

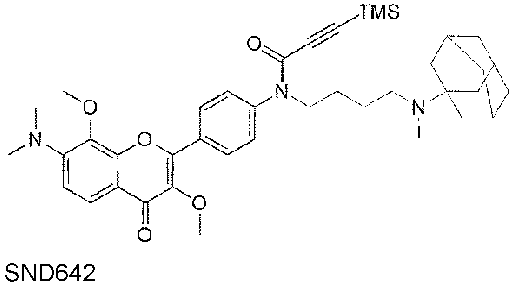

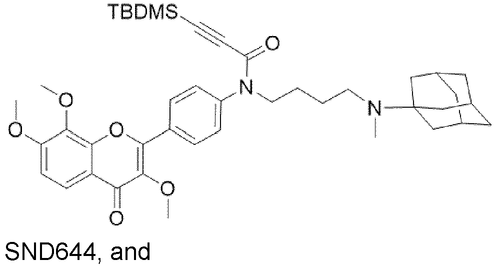

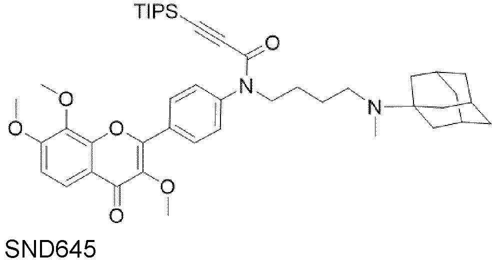

In a preferred embodiment, the compound of general formula (I) is selected from SND462AM, SND462AMH, SND630, SND631, SND633, SND635, SND642, SND644, and SND645.

The compound described herein in its free from or in its pharmaceutically acceptable form may be anhydrous or in the form of a hydrate (e.g., a hemihydrate, monohydrate, dihydrate or trihydrate) or other solvate. Such solvates may be formed with common organic solvents, including but not limited to, alcoholic solvents e.g., methanol, ethanol or isopropanol.

The compound described herein in its free from or in its pharmaceutically acceptable form may be in any polymorphic or amorphous form.

A **second** aspect according to the present invention is directed to a pharmaceutical composition containing the compound described herein and one or more excipients.

Conventional procedures for the selection and preparation of suitable pharmaceutical formulations are described in, for example, "Aulton's Pharmaceutics - The Design and Manufacture of Medicines", M. E. Aulton and K. M. G. Taylor, Churchill Livingstone Elsevier, 4th Ed., 2013.

The one or more excipients may be selected from fillers, binders, ionic strength adjusting media, complexing agents, disintegrants, flavorants, and sweeteners.

Examples of suitable fillers include, but are not limited to starches, lactose, mannitol, Pearlitol^{™} SD 200, cellulose derivatives, sugar, and the like.

Examples of suitable binders include, but are not limited to, hydroxypropylcellulose (Klucel^{™}-LF), hydroxypropyl methylcellulose or hypromellose (Methocel^{™}), polyvinylpyrrolidone or povidone (PVP-K25, PVP-K29, PVP-K30, PVP-K90), plasdone S 630 (copovidone), powdered acacia, gelatin, guar gum, carbomer (e.g., carbopol), methylcellulose, polymethacrylates, and starch.

Suitable ionic strength adjusting media include buffers and salts, e.g., phosphate buffer, such as phosphate-buffered-saline, isotonic NaCl solution, sugar solution, citrate buffer, isocitrate buffer, EDTA (including EDTA salts), and mixtures thereof.

Examples of suitable disintegrants include, but are not limited to carmellose calcium, carboxy methylstarch sodium, croscarmellose sodium, crospovidone, and low-substituted hydroxypropylcellulose. The powdered formulation may further comprise an anti-adherent agent, such as, for example, talc, silica derivatives, or silicon dioxide.

The pharmaceutical composition may be administered by oral, parental (including intravenous, subcutaneous, intramuscular, intradermal, intratracheal, intraperitoneal, intraarticular, intracranial and epidural), airway (aerosol), rectal, vaginal or topical (including transdermal, buccal, mucosal and sublingual) administration.

In one preferred embodiment the pharmaceutical composition is adapted to oral administration. For oral administration, the compound may be incorporated with suitable excipients and used in the form of ingestible tablets, buccal tablets, troches, capsules, elixirs, suspensions, syrups, wafers, and the like. The percentage of the therapeutic compound in the compositions may, of course, be varied. The amount of the compound in such therapeutically useful compositions is such that a suitable dosage will be obtained.

Formulations for oral administration may be specifically formulated for pediatric use. The compound may be prepared as a powder, which may be packaged in individual (i.e., single-use) unit doses. The powder formulation may be in granulated form having, for example, a particle size within the range of about 1 µM to about 1000 µM in diameter. Individual unit doses may be in the form of sachets, capsules, etc. The powdered therapeutic compound in an individual unit dose may be formulated with one or more excipients, such as fillers, binders, complexing agents, disintegrants, flavorants, and sweeteners. The powdered formulation may further comprise an anti-adherent agent, such as, for example, talc, silica derivatives, or silicon dioxide. The powder pharmaceutical composition may be administered as a powder, or may be reconstituted in food or drink prior to oral administration.

The pharmaceutical composition for oral administration may be a liquid concentrate containing one or more of the fillers described herein, which may be packaged in individual (i.e., single-use) unit dose or in multi-use dose formats. Concentrated liquid formulations may comprise the therapeutic agent and a solvent (e.g., an organic or aqueous solvent). Concentrated liquid formulations include solutions, syrups, etc. A concentrated liquid formulation may comprise a component to mask the taste of the therapeutic compound. A liquid composition for oral administration may be obtainable by mixing the concentrated liquid formulation with an aqueous medium. A multi-use dose format of concentrated liquid may be provided with an at-home dispending method.

The pharmaceutical composition may be a tablet coated for paediatric administration containing one or more of the fillers described herein. A tablet may be scored. A scored table may comprise a single unit dose or multiple unit doses. A scored multiple unit dose tablet may be converted into single unit doses by cutting along said scoring. In some aspects, a tablet will readily dissolve in water for liquid administration.

The pharmaceutical composition may be prepared in a chewable form. Such a chewable form may be soft (e.g., gelatinous) or hard. The chewable form may comprise a chewable base(s) (e.g., xylitol, mannitol and sorbitol), complexing agent(s), binder(s) (e.g, hydroxypropyl cellulose, polyvinylpyrrolidone, pregelatinized starch and the like), disintegrants (e.g., crospovidone, sodium starch glycolate, starches such as maize starch and dried starch, croscarmellose sodium and cellulose products such as microcrystalline cellulose, microfine cellulose, low substituted hydroxypropylcellulose), lubricants (e.g., magnesium stearate, colloidal silicon dioxide and the like), sweetening agents (e.g., natural sweeteners such as sugars and artificial sweetening agents such as sodium saccharin or aspartame), coloring agents, and flavoring agents (e.g, fruit flavours, which may be natural or synthetic). Any of the foregoing formulations may be stable a room temperature. Any of the foregoing formulations may be stable at about 4°C.

In a further preferred embodiment, the pharmaceutical composition is adapted to a parenteral administration, preferably to an intravenous administration. Dispersions can be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms, such as bacteria and fungi.

Based on the present disclosure, suitable dosage forms and dosage levels can be selected by those of ordinary skill in the art for a particular patient. As the skilled person understands, the dose may be dependent on the route of administration. In addition, lower or higher doses may be required depending on particular factors. For instance, specific doses and treatment regimens will depend on factors such as the patient's general health profile, and the severity and course of the patient's disorder or disposition thereto.

A **third** aspect according to the present invention is directed to a compound as claimed and described herein, and a pharmaceutical composition as claimed and described herein for use as a medicament.

A **fourth** aspect according to the present invention is directed to a compound as claimed and described herein, and a pharmaceutical composition as claimed and described herein for use in the treatment or prevention of cancer including therapy-resistant cancer and rare cancer forms.

As used herein, the term "treatment" refers equally to curative therapy, and ameliorating or palliative therapy. The term includes obtaining beneficial or desired physiological results, which may or may not be established clinically. Beneficial or desired clinical results include, but are not limited to, the alleviation of symptoms, the prevention of symptoms, the diminishment of extent of disease, the stabilisation (i.e., not worsening) of a condition, the delay or slowing of progression/worsening of a condition/symptoms, the amelioration or palliation of the condition/symptoms, and remission (whether partial or total), whether detectable or undetectable. The term "palliation", and variations thereof, as used herein, means that the extent and/or undesirable manifestations of a physiological condition or symptom are lessened and/or time course of the progression is slowed or lengthened, as compared to not administering a compound, multi-salt, solvate, or pharmaceutical composition of the present invention.

The term "prevention" as used herein in relation to a disease, disorder or condition, relates to prophylactic or preventative therapy, as well as therapy to reduce the risk of developing the disease, disorder or condition. The term "prevention" includes both the avoidance of occurrence of the disease, disorder or condition, and the delay in onset of the disease, disorder or condition. Any statistically significant avoidance of occurrence, delay in onset or reduction in risk as measured by a controlled clinical trial may be deemed a prevention of the disease, disorder or condition. Subjects amenable to prevention include those at heightened risk of a disease, disorder or condition as identified by genetic or biochemical markers.

Advantageously the compound described herein proved efficient in a variety of different cancer types, including therapy-resistant (e.g., chemotherapy-resistant, targeted-therapy resistant, hormone-therapy resistant, radiation-therapy resistant) cancer forms and rare cancer forms. In one embodiment, the cancer is selected from, but not limited to:
- brain cancer,
- bladder cancer,
- breast cancer,
- hematologic cancer,
- head and neck cancer,
- intestinal cancer,
- kidney cancer,
- skin cancer,
- gynecologic cancer,
- pancreatic cancer,
- prostate cancer,
- lung cancer,
- liver cancer,
- stomach cancer,
- soft tissue cancer,
- esophageal cancer,
- testicular cancer,
- bone cancer, and
- thyroid cancer.

In one embodiment, the cancer is brain cancer. The brain cancer may be a therapy-resistant cancer and/or a rare brain cancer form. The brain cancer includes, but is not limited to: brain neuroblastoma, brain glioblastoma and medulloblastoma, including desmoplastic cerebellar medulloblastoma.

In one embodiment, the cancer is bladder cancer. The bladder cancer may be a therapy-resistant cancer and/or a rare cancer form. The bladder cancer includes, but is not limited to: bladder carcinoma.

In one embodiment, the cancer is breast cancer. The breast cancer may be a therapy-resistant cancer and/or a rare cancer form. The breast cancer includes but is not limited to: breast ductal carcinoma, including invasive breast ductal carcinoma and breast ductal carcinoma grade 3, breast pleomorphic carcinoma, breast squamous cell carcinoma, and metastatic adenocarcinoma.

In one embodiment, the cancer is hematologic cancer, in particular blood leukemia, blood lymphoma and blood myeloma. The hematologic cancer may be a therapy-resistant cancer and/or a rare cancer form.

Blood leukemia includes, but it is not limited to: acute myeloid leukemia (AML), chronic myeloid leukemia (CML), acute lymphoblastic leukemia (ALL) including B-cell and T-cell, and chronic eosinophilic leukemia.

Blood lymphoma includes, but it is not limited to: Hodgkin lymphoma and non-Hodgkin lymphoma, in particular non-Hodgkin lymphoma, including B-cell lymphoma such as, Burkitt lymphoma, diffuse large B-cell lymphoma, and mantel cell lymphoma, and T-cell lymphoma such as, cutaneous T-cell lymphoma, and anaplastic large cell lymphoma.

Blood myeloma includes but is not limited to: plasmacytoma and multiple myeloma.

In one embodiment, the cancer is head and neck cancer. The head and neck cancer may be a therapy-resistant cancer and/or a rare cancer form. The head and neck cancer includes but is not limited to: tongue squamous cell carcinoma and pharynx squamous cell carcinoma.

In one embodiment, the cancer is intestinal cancer. The intestinal cancer may be a therapy-resistant cancer and/or a rare cancer form. The intestinal cancer includes but is not limited to:
- duodenal cancer, in particular duodenal adenocarcinoma,
- colorectal cancer in particular colorectal adenocarcinoma including Duke's type D and Duke's type C,
- colon carcinoma including colon adenocarcinoma,
- rectal carcinoma, in particular rectal adenocarcinoma, and
- cecum carcinoma, in particular cecum adenocarcinoma.

In one embodiment, the cancer is the pancreatic cancer, in particular pancreatic adenocarcinoma (ductal adenocarcinoma). The pancreatic cancer may be a therapy-resistant cancer and/or a rare cancer form.

In one embodiment, the cancer is liver cancer, in particular hepatocarcinoma. The liver cancer may be a therapy-resistant cancer and/or a rare cancer form.

In one embodiment, the cancer is skin cancer, in particular skin melanoma. The skin cancer may be a therapy-resistant cancer and/or a rare cancer form.

In one embodiment, the cancer is lung cancer. The lung cancer may be a therapy-resistant cancer and/or a rare cancer form. The lung cancer includes but is not limited to: small cell lung carcinoma, carcinoid, pleural mesothelioma, and non-small cell lung carcinoma, in particular adenocarcinoma, large cell carcinoma, squamous cell carcinoma and bronchoalveolar carcinoma.

In one embodiment, the cancer is gynecologic cancer. The gynecologic cancer may be a therapy-resistant cancer and/or a rare cancer form. The gynecologic cancer includes but is not limited to:
- cervical cancer,
- uterine/endometrial cancer, in particular endometrial adenocarcinoma and uterine choriocarcinoma, and
- ovarian cancer, in particular ovarian teratocarcinoma and ovarian adenocarcinoma including ovarian clear-cell adenocarcinoma, ovarian serous cyst adenocarcinoma including high grade ovarian serous adenocarcinoma, ovarian endometrioid adenocarcinoma, ovarian primary malignant adenocarcinoma, and ovarian serous cystadenocarcinoma.

In one embodiment, the cancer is stomach cancer. The stomach cancer may be a therapy-resistant cancer and/or a rare cancer form. The stomach cancer includes but is not limited to: stomach carcinoma, in particular adenosquamous carcinoma and cholangiocellular carcinoma, and stomach adenocarcinoma including signet ring cell stomach adenocarcinoma.

In one embodiment, the cancer is soft tissue (muscle) cancer. The soft tissue (muscle) cancer may be a therapy-resistant cancer and/or a rare cancer form. The soft tissue (muscle) cancer includes but is not limited to: fibrosarcoma, rhabdomyosarcoma including embryonal rhabdomyosarcoma, liposarcoma, biphasic synovial sarcoma, epithelioid sarcoma and Ewig sarcoma.

In one embodiment, the cancer is esophageal cancer, in particular esophageal squamous cell carcinoma. The esophageal cancer may be a therapy-resistant cancer and/or a rare cancer form.

In one embodiment, the cancer is testicular cancer, in particular testicular embryonal carcinoma. The testicular cancer may be a therapy-resistant cancer and/or a rare cancer form.

In one embodiment, the cancer is bone cancer, in particular osteosarcoma or Ewing sarcoma. The bone cancer is usually a therapy-resistant cancer and/or a rare cancer form.

In one embodiment, the cancer is thyroid cancer, in particular hereditary thyroid gland medullary carcinoma or anaplastic carcinoma. The thyroid cancer may be as well therapy-resistant cancer and/or a rare cancer form.

### EXAMPLES

To further illustrate the invention, the following examples are provided. These examples are provided with no intend to limit the scope of the invention.

### A. Preparation of compounds according to the present invention

### General Experimental Details

All chemicals used were reagent grade and used as supplied, unless noted otherwise.

Unless otherwise indicated, all liquid chromatography-mass spectrometry-mass (LC-MS) data (sample analyzed for purity and identity) were obtained with a Shimadzu model-LC-40AD system using an Shimadzu model LCMS-2020 mass spectrometer utilizing ESI ionization fitted with an HALO (C18 3.0X30mm, 5µm) reverse-phase column at 50°C. The mobile phase consisted of a mixture of solvent 0.04% trifluoroacetic acid in water and 0.02% trifluoroacetic acid in MeCN.

1H NMR was recorded on a Bruker Avance Neo (400 MHz) spectrometer in DMSO-d6 or chloroform-d with chemical shifts referenced to internal standards unless stated otherwise. Splitting patterns are indicated as s, singlet; d, doublet; t, triplet; q, quartet; m, multiplet; bs, broad singlet for 1H-NMR data. NMR chemical shifts (δ) are reported in ppm and coupling constants (J) are reported in Hz.

Unless otherwise indicated, all HPLC purifications were conducted using a Phenomenex luna C18 150*25 mm* 10µm column with a mobile phase: [H₂O (0.225% FA) -ACN] and a gradient:32%-62% B over 12.0 min.

### A.1 Synthesis of compounds SND462-AM and SND462-AMH

The compound SND462-AM was synthesized following the reaction sequence in Scheme 1.

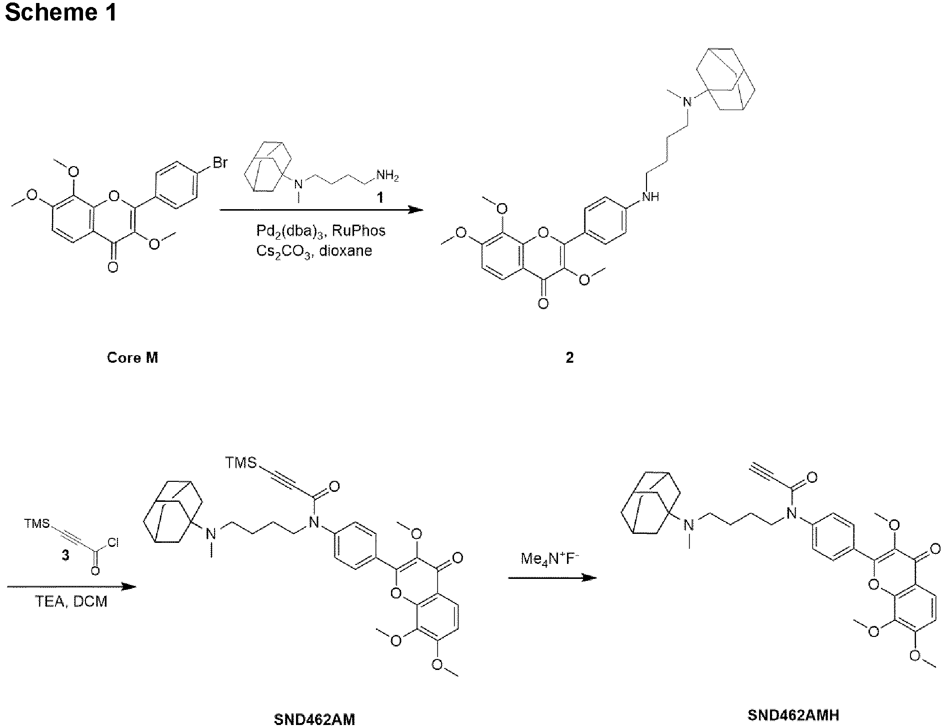

### A.1.1 Synthesis of compound 2

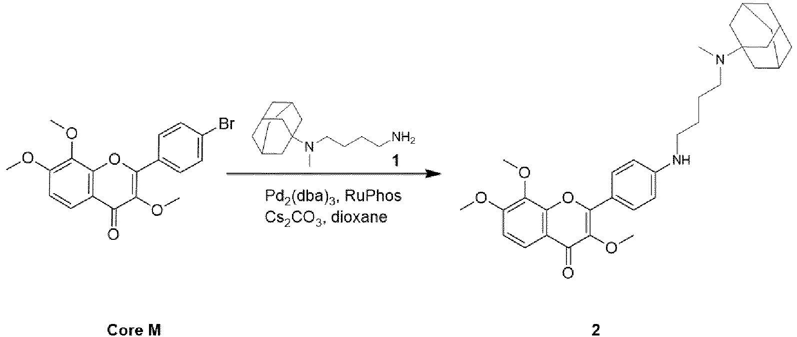

A suspension of **Core M** (500 mg, 1.28 mmol, 1 eq), **compound 1** (362.56 mg, 1.53 mmol, 1.2 eq), Pd₂(dba)₃ (117.04 mg, 127.81 µmol, 0.1 eq), RuPhos (59.64 mg, 127.81 µmol, 0.1 eq), Cs₂CO₃ (1.25 g, 3.83 mmol, 3 eq) in DMF (5 mL) was stirred at 80 °C for 3 h under N₂ (10 batches in parallel). The reaction mixture was filtered and concentrated under reduced pressure to give a residue. The crude product was purified by reversed-phase HPLC (0.1% FA). **Compound 2** (5 g, 11.89 mmol, 71.56% yield, 99% purity) was obtained as a yellow solid.
**LCMS:** MS (ESI) Retention time: 0.478 min, (M+1) ⁺ =547.3

### A.1.2 Synthesis of compound SND462AM

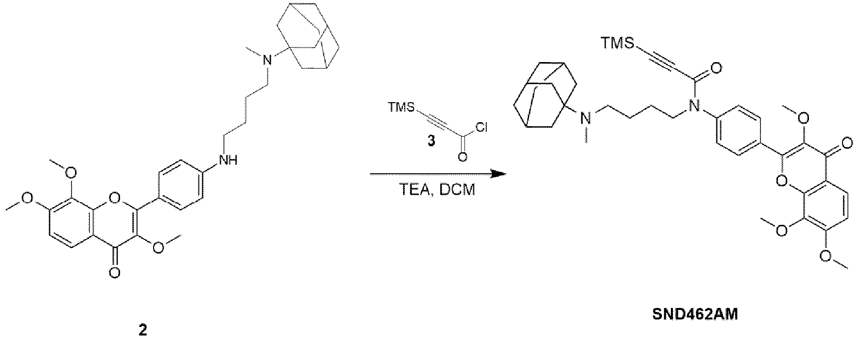

To a solution of **compound 2** (1.25 g, 2.29 mmol, 1 eq), triethylamine (542.58 mg, 6.86 mmol, 553.65 µL, 3 eq) in THF (12 mL) was added **compound 3** (551.06 mg, 3.43 mmol, 1.5 eq), and then the mixture was stirred at 0 °C for 10min (4 batches in parallel). The reaction mixture was washed with ice water (30 ml × 2), and extracted with DCM (20 ml × 3), the organic phase was washed with NaHCO₃ (20 ml × 2), and then the organic phase was washed with H₂O (20 ml × 3), the organic phase was dried over Na₂SO₄, filtered and concentrated under reduced pressure to give a residue. **SND 462AM** (5.6 g, 4.44 mmol, 88.52% yield, 96.99% purity) was obtained as a yellow solid.
**LCMS:** MS (ESI) Retention time: 0.533 min, (M+1)⁺ =671.5
**¹H NMR** (400 MHz, CHLOROFORM-d) δ = 8.23-8.21 (d, J = 8.4 Hz, 2H), 8.02-8.00 (d, J = 9.2 Hz, 1H), 7.46-7.44 (d, J = 8.4 Hz, 2H), 7.09-7.07 (d, J = 9.2 Hz, 1H), 4.02 (s, 6H), 3.94 (s, 3H), 3.88 - 3.82 (m, 1H), 3.76-3.75 (t, J = 6.4 Hz, 1H), 2.12-2.10 (br d, J = 8.4 Hz, 3H), 1.867-1.85(td, J = 3.2, 6.4 Hz, 2H), 1.61 (br s, 19H), 0.19 - 0.04 (m, 9H).

### A.2 Synthesis of compound SND462AMH

To a solution of SND462AM (1.5 g, 2.24 mmol, 1 eq) in THF (10 mL) was added Me₄N⁺F⁻(166.60 mg, 1.79 mmol, 0.8 eq), and then the mixture was stirred at 25 °C for 2h. TLC (SiO₂, DCM/MeOH=10/1, Rf = 0.6) showed SND462AM was consumed and a new spot detected. The reaction mixture was concentrated under reduced pressure to give a residue. The residue was purified by column chromatography (Al₂O3, petroleum ether/ethyl acetate=50/1 to 1/1 to 0/1). SND 462AMH (1.5 g, 2.92 mmol, 54.5% yield, 97.27% purity) was obtained as a light yellow solid.
**LCMS:** MS (ESI) Retention time: 0.509 min, (M+1) + =599.4
**¹H NMR** (400 MHz, CHLOROFORM-d) δ = 8.28-8.26 (br d, J = 8.8 Hz, 2H), 8.01-7.99 (d, J = 9.2 Hz, 1H), 7.46-7.44 (d, J = 8.8 Hz, 2H), 7.09-7.07 (d, J = 9.2 Hz, 1H), 4.05-4.03 (d, J = 7.6 Hz, 6H), 3.98 - 3.93 (m, 3H), 3.86-3.84 (br t, J = 7.6 Hz, 2H), 2.86 (s, 1H), 2.43 - 2.35 (m, 2H), 2.20 (br s, 3H), 2.05 (s, 4H), 1.62 - 1.54 (m, 14H), 1.51 - 1.44 (m, 2H).

### A.3 Synthesis of compound SND462AMM

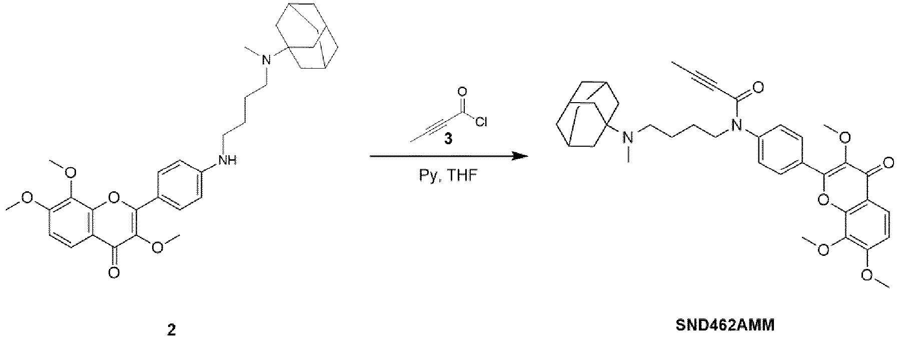

To a solution of **compound 2** obtained as described at section A.1.1 (100 mg, 182.92 µmol, 1 eq) in THF (1 mL) was added pyridine (43.41 mg, 548.75 µmol, 44.29 µL, 3 eq), the mixture was stirred at 0 °C for 5 min, then the mixture was added **compound** 3 (28.13 mg, 274.38 µmol, 1.5 eq) at 0 °C, the mixture was stirred at 0 °C for 5 min. The mixture was concentrated at reduced pressure at 25 °C. The residue was poured into water (10 mL). The aqueous phase was extracted with DCM (10 mL × 2). The combined organic phase was poured into aq. NaHCO₃ (10 mL), the aqueous phase was extracted with DCM (10 mL × 2), The combined organic phase was poured into aq. Citric acid (10 mL), the aqueous phase was extracted with DCM (10 mL × 2), The combined organic phase was poured into water (10 mL). The aqueous phase was extracted with DCM (10 mL × 2) dried with anhydrous Na₂SO₄, filtered and concentrated in vacuum. **SND462AMM** (113.23 mg, crude) was obtained as yellow solid.
**LCMS:** MS (ESI) Retention time: 0.490 min, (M+1)⁺ =613.4
**¹H NMR** (400 MHz, DMSO-d6) δ = 8.12 (br d, J = 6.8 Hz, 2H), 7.83 (d, J = 9.0 Hz, 1H), 7.66 - 7.51 (m, 2H), 7.31 (d, J = 9.2Hz, 1H), 3.95 (d, J = 18.6 Hz, 7H), 3.88 - 3.79 (m, 3H), 3.79 - 3.73 (m, 1H), 2.41 (td, J = 4.2, 10.4 Hz, 1H), 2.27 - 2.06 (m, 4H), 2.00 (br s, 3H), 1.83 - 1.34 (m, 19H).

### A.4 Synthesis of compounds SND630 and SND631

The compounds SND630 and SND631 was synthesized following the reaction sequence depicted in Scheme 2.

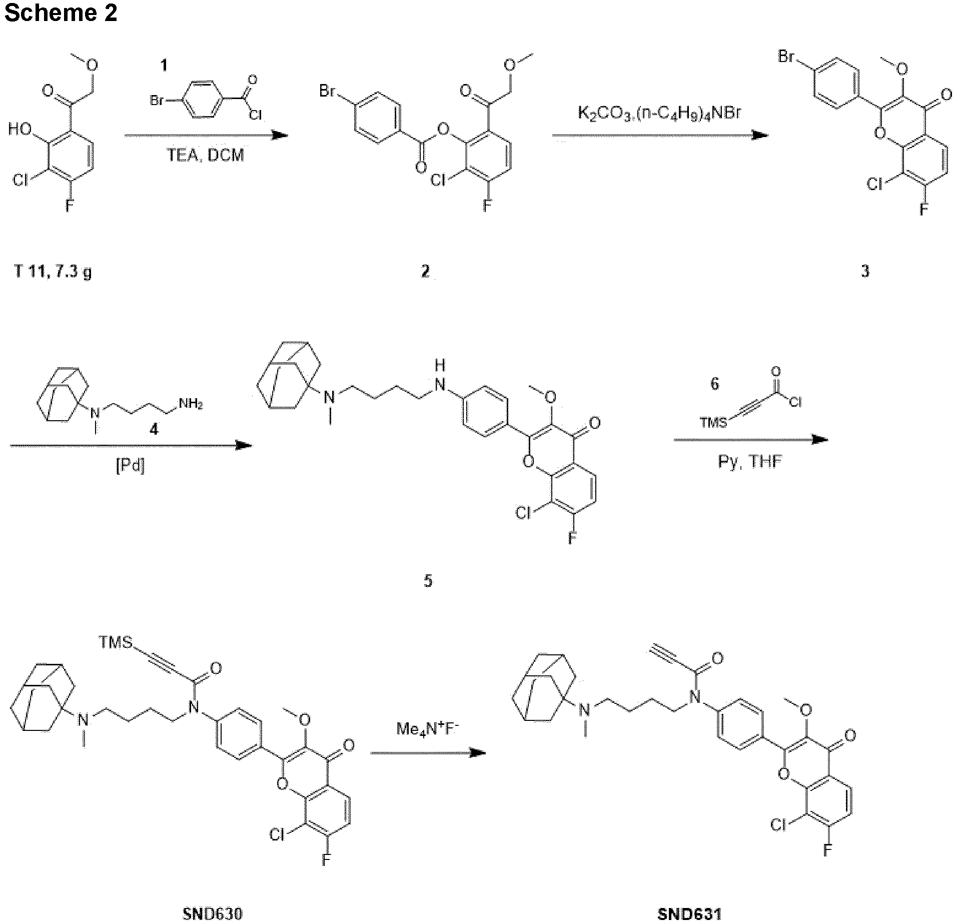

### A.4.1 Synthesis of compound 2

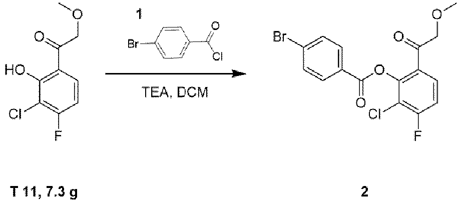

To a solution of **compound T11** (2 g, 9.15 mmol, 1 eq), triethylamine (2.17 g, 27.45 mmol, 2.22 mL, 3 eq) in DCM (20 mL) was added **compound** 1 (2.41 g, 10.98 mmol, 1.2 eq) at 0 °C, and then the mixture was stirred at 25 °C for 3h. TLC (SiO₂, petroleum ether / ethyl acetate = 3/1, Rf=0.6) showed **compound T11** was consumed and a new spot detected. The mixture was poured into H₂O (30ml), extracted with ethyl acetate (20 mL × 2). The combined organic layers were washed with brine (20 mL), dried over Na₂SO₄, filtered and concentrated under reduced pressure to give compound 2 as the crude product and the crude product was used to the next step directly.
**LCMS:** MS (ESI) Retention time: 0.688 min, (M+1) ⁺ =433.0
**¹H NMR** (400 MHz, CHLOROFORM-d) δ = 8.10-8.08 (d, J = 8.8 Hz, 2H), 7.84-7.82 (dd, J = 5.6, 8.8 Hz, 1H), 7.72-770 (d, J = 8.4 Hz, 2H), 7.24-7.22 (t, J = 8.4 Hz, 1H), 4.47 (s, 2H), 3.38 (s, 3H).

### A.4.2 Synthesis of compound 3

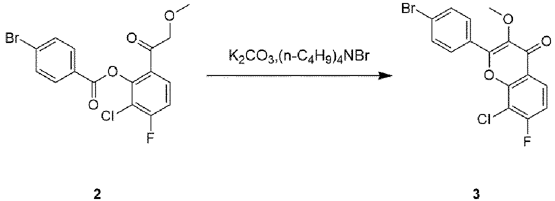

To a solution of **compound 2** (3.5 g, 8.71 mmol, 1 eq), K₂CO₃ (7.23 g, 52.29 mmol, 6 eq), TBAB (4.21 g, 13.07 mmol, 1.5 eq) in toluene (30 mL), and then stirred at 110 °C for 12h under N₂. TLC (SiO₂, petroleum ether / ethyl acetate =3/1 , Rf = 0.4) showed **compound 2** was consumed and some new spots detected. The reaction mixture was poured into H₂O (30 ml), and added citric acid to pH = 7, and extracted with ethyl acetate (20 ml × 3), dried over Na₂SO₄, filtered and concentrated under reduced pressure to give a residue. The residue was purified by column chromatography (SiO₂, Petroleum ether/Ethyl acetate=50/1 to 3/1). And then triturated with MeOH 10ml. **Compound 3** (450 mg, 1.13 mmol, 12.92% yield, 96% purity) was obtained as an off-white solid.
**LCMS:** MS (ESI) Retention time: 0.650 min, (M+1)⁺ =385.0
**¹H NMR** (400 MHz, CHLOROFORM-d) δ = 8.19-8.17 (dd, J = 6.0, 8.8 Hz, 1H), 8.13 - 8.08 (m, 2H), 7.90-7.88 (dd, J = 1.6, 8.4 Hz, 1H), 7.72 - 7.67 (m, 2H), 7.65 - 7.59 (m, 1H), 7.27 - 7.22 (m, 1H), 3.94 (s, 3H).

### A.4.3 Synthesis of compound 5

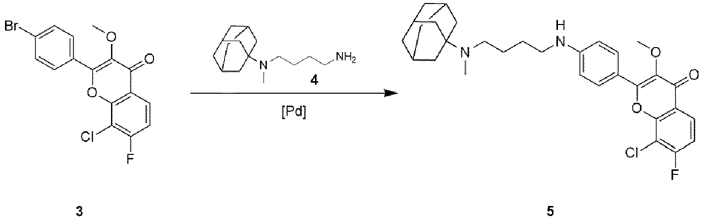

To a mixture of **compound 3** (80 mg, 208.55 µmol, 1 eq), **compound 4** (49.30 mg, 208.55 µmol, 1 eq), Cs₂CO₃ (203.85 mg, 625.66 µmol, 3 eq) in dioxane (1 mL) was added RuPhos-Pd-G4 (17.74 mg, 20.86 µmol, 0.1 eq), and then the mixture was stirred at 90 °C for 16h under N2. The reaction mixture was filtered and concentrated under reduced pressure to give a residue. The residue was purified by prep-HPLC. **Compound 5** (100 mg, 185.50 µmol, 88.95% yield) was obtained as a yellow solid.
**LCMS:** MS (ESI) Retention time: 0. 571 min, (M+1) ⁺ =539.3

### A.4.4 Synthesis of compound SND630

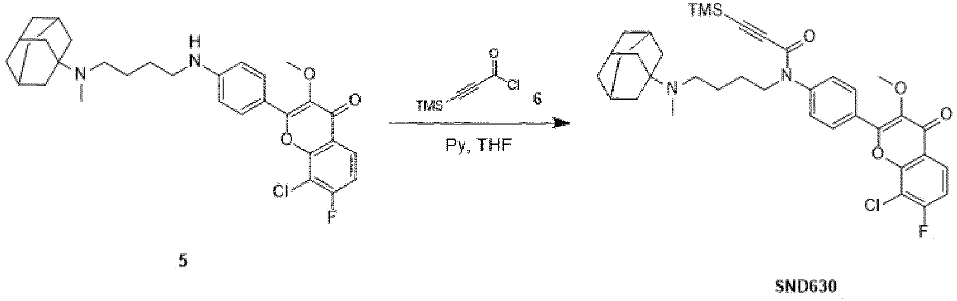

To a solution of **compound 5** (110 mg, 204.05 µmol, 1 eq), pyridine (48.42 mg, 612.16 µmol, 49.41 µL, 3 eq) in THF (0.1 mL) was added **compound 6** (49.18 mg, 306.08 µmol, 1.5 eq) at 0 °C, and then the mixture was stirred at 25 °C for 0.5 h. The reaction mixture was concentrated under reduced pressure to give a residue. It was used into the next step directly. **SND630** (130 mg, 195.99 µmol, crude) was obtained as a yellow solid.
**LCMS:** MS (ESI) Retention time: 0. 586 min, (M+1) ⁺ =663.3

### A.4.5 Synthesis of compound SND631

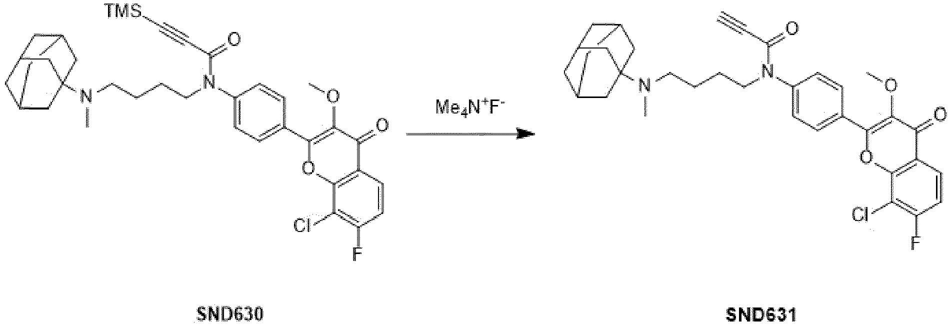

To a solution of **SND630** (130 mg, 195.99 µmol, 1 eq) in THF (0.1 mL) was added Me₄N⁺F⁻(14.60 mg, 156.79 µmol, 0.8 eq), and then the mixture was stirred at 25 °C for 2h. TLC (SiO₂, DCM/MeOH=10/1, Rf = 0.5) showed **SNS630** was consumed and a new spot detected. The reaction mixture was concentrated under reduced pressure to give a residue. The residue was purified by column chromatography (Al₂O₃, Petroleum ether/Ethyl acetate=50/1 to 3/1). **SND631** (90 mg, 141.28 µmol, 72.08% yield, 92.79% purity) was obtained as a yellow oil.
**LCMS:** MS (ESI) Retention time: 0. 508min, (M+1)⁺ =519.5
**¹H NMR** (400 MHz, CHLOROFORM-d) δ = 8.32-8.30 (br d, J = 8.4 Hz, 2H), 8.19-8.17 (dd, J = 6.0, 8.8 Hz, 1H), 7.48-7.46 (d, J = 8.8 Hz, 2H), 7.26 - 7.19 (m, 1H), 4.02 - 3.94 (m, 3H), 3.94 - 3.80 (m, 2H), 2.89 - 2.82 (m, 1H), 2.40-2.38 (br t, J = 7.2 Hz, 2H), 2.19 (s, 3H), 2.05 (br s, 3H), 1.65 - 1.55 (m, 14H), 1.49-1.47 (br d, J = 5.6 Hz, 2H).

### A.5 Synthesis of compounds SND635 and SND642

Compounds SND635 and SND642 were synthesized following the reaction sequence depicted in Scheme 3.

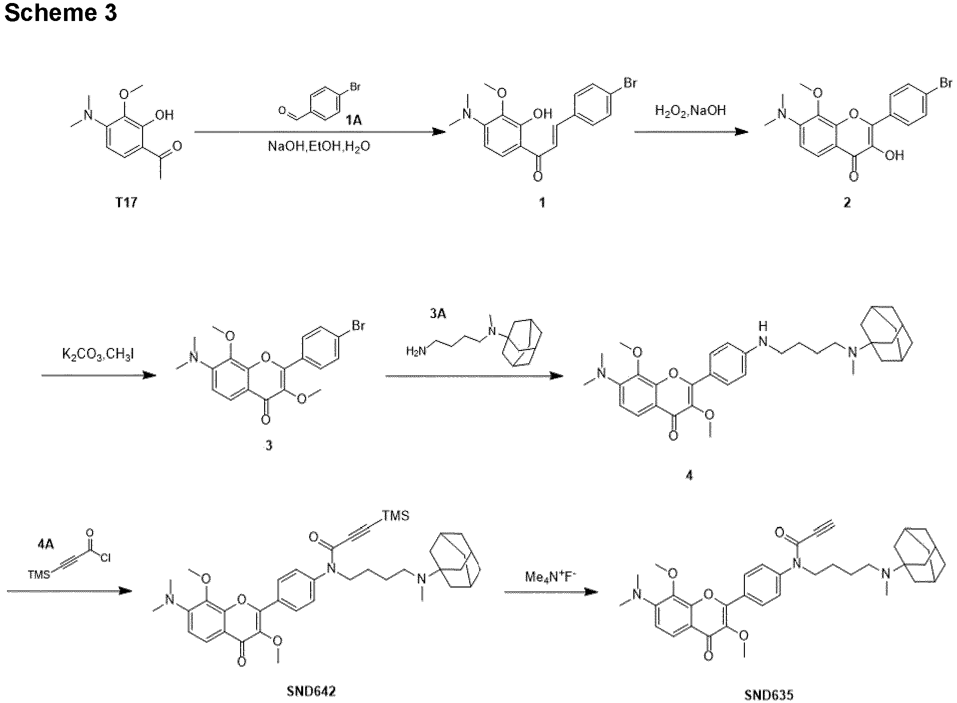

### A.5.1 Synthesis of compound 1

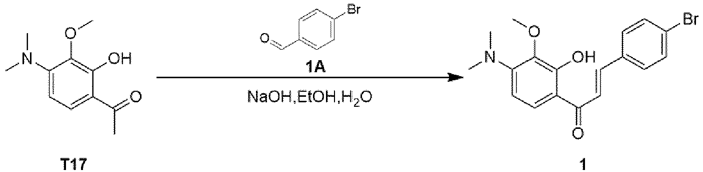

A mixture of **compound T17** (1 g, 4.78 mmol, 1 eq), **compound 1A** (1.77 g, 9.56 mmol, 2 eq), KOH (1.34 g, 23.88 mmol, 5.00 eq) in EtOH (10 mL) and H₂O (2.5 mL), and the mixture was stirred at 80 °C for 30min under microwave (3 batch). TLC (SiO₂, Petroleum ether/Ethyl acetate= 3/1, Rf = 0.6) showed **compound T17** was consumed and a new spot detected. The reaction mixture was added 1 M HCl to pH=6, and then extracted with ethyl acetate (50 ml × 3), and the organic phase was dried over Na₂SO₄, concentrated under reduced pressure to give a residue. The residue was purified by column chromatography (SiO₂, Commercial hexanes: Ethyl acetate=50/1 to 4/1) to give a crude product. The crude product was purified by re-crystallization from MeOH (30 ml). **Compound** 1 (2.5 g, 6.25 mmol, 43.56% yield, 94% purity) was obtained as a yellow solid.
**LCMS:** MS (ESI) Retention time: 0.679 min, (M+1) ⁺ =376.1
**¹H NMR** (400 MHz, CHLOROFORM-d) δ = 13.45 (s, 1H), 7.80-7.78 (d, J = 15.2 Hz, 1H), 7.59 - 7.49 (m, 6H), 6.36-6.34 (d, J = 9.2 Hz, 1H), 3.81 (s, 3H), 3.11 (s, 6H).

### A.5.2 Synthesis of compound 2

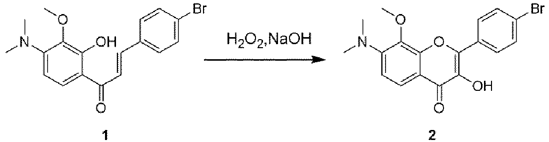

To a solution of **compound 1** (1 g, 2.66 mmol, 1 eq) in MeOH (10 mL) was added a solution of NaOH (425.23 mg, 10.63 mmol, 4 eq) in H₂O (1 mL), and then the mixture was cooled to 0 °C, and H₂O₂ (7.800 g, 68.79 mmol, 6.61 mL, 30% purity, 25.88 eq) was added slowly, and then the mixture was warmed to 25 °C, and stirred for another 2 h. TLC (SiO₂, Petroleum ether/Ethyl acetate = 3/1, Rf = 0.3) showed **compound 1** was consumed and a new spot detected. The reaction mixture was poured into saturated Na₂SO₃ (100 ml) slowly at 0°C, and then added HCl (2M) to pH=6 at 0 °C, extracted with DCM (40 ml × 3) and H₂O (20 ml × 3), the organic phase was dried over Na₂SO₄, filtered and concentrated under reduced pressure to give a residue. The residue was purified by column chromatography (SiO₂, Commercial hexanes: Ethyl acetate=50/1 to 3/1). **Compound 2** (300 mg, 691.90 µmol, 26.03% yield, 90% purity) was obtained as a yellow solid.
**LCMS:** MS (ESI) Retention time: 0.590 min, (M+1) ⁺ =392.1
**¹H NMR** (400 MHz, CHLOROFORM-d) δ = 8.19-8.17 (d, J = 8.8 Hz, 2H), 7.86-7.84 (d, J = 9.2 Hz, 1H), 7.66-7.64 (d, J = 8.8 Hz, 2H), 6.93-6.91 (d, J = 9.2 Hz, 1H), 3.91 (s, 3H), 3.10 (s, 6H)

### A.5.3 Synthesis of compound 3

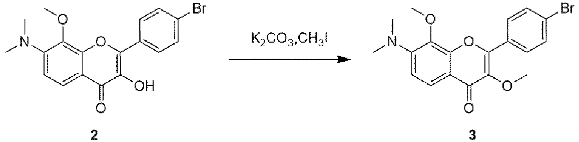

To a solution of **compound 2** (250 mg, 640.65 µmol, 1 eq), K₂CO₃ (221.35 mg, 1.60 mmol, 2.5 eq) in DMF (3 mL) was added CH₃I (136.40 mg, 960.98 µmol, 59.82 µL, 1.5 eq) at 0 °C slowly and then the mixture was stirred at 25 °C for 2h. TLC (SiO₂, petroleum ether/ethyl acetate = 3/1, 0.4) showed **compound 1** was consumed and a new spot detected. The reaction mixture was washed with H₂O (20 ml × 2) and ethyl acetate (20 ml × 2), and the organic phase was dried over Na₂SO₄, filtered and concentrated under reduced pressure to give a residue. The residue was purified by column chromatography (SiO₂, Commercial hexanes: Ethyl acetate=50/1 to 4/1). **Compound 3** (210 mg, 488.31 µmol, 76.22% yield, 94% purity) was obtained as a yellow solid.
**LCMS:** MS (ESI) Retention time: 0.653 min, (M+1) ⁺ =406.0

### A.5.4 Synthesis of compound 4

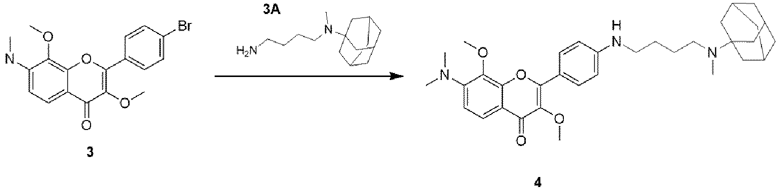

To a solution of **Compound 3** (90 mg, 222.63 µmol, 1 eq), **compound 3A** (63.16 mg, 267.16 µmol, 1.2 eq), Gphos-Pd-G6 (21.03 mg, 22.26 µmol, 0.1 eq) in dioxane (2 mL) was added t-BuONa (2 M, 166.97 µL, 1.5 eq), and then the mixture was degassed and purged with N₂ for 3 times, the mixture was stirred at 65 °C for 12h under N₂. The reaction mixture was filtered and concentrated under reduced pressure to give a residue. The residue was purified by prep-HPLC. **Compound 4** (100 mg, crude) was obtained as a yellow solid.
**LCMS:** MS (ESI) Retention time: 0.483 min, (M+1) ⁺ =560.4

### A.5.5 Synthesis of compound SND642

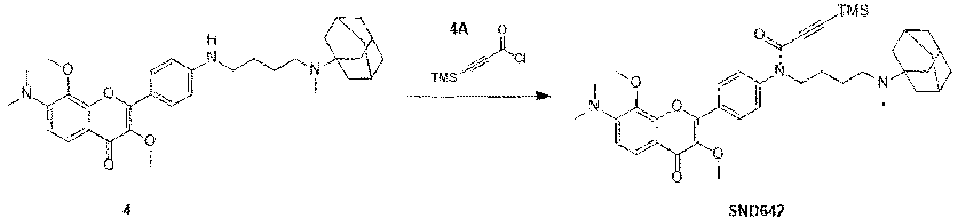

To a solution of **compound** 4 (80 mg, 142.92 µmol, 1 eq), pyridine (33.92 mg, 428.77 µmol, 34.61 µL, 3 eq) in THF (2 mL) was added **compound 4A** (34.45 mg, 214.39 µmol, 1.5 eq) at 0 °C, and then the mixture was stirred at 25 °C for 0.5 h. The reaction mixture was concentrated under reduced pressure to give a residue. It was used into the next step without purification. **SND642** (180 mg, crude) was obtained as a yellow solid.
**LCMS:** MS (ESI) Retention time: 0.553 min, (M+1) ⁺ =684.4

### A.5.6 Synthesis of compound SND635

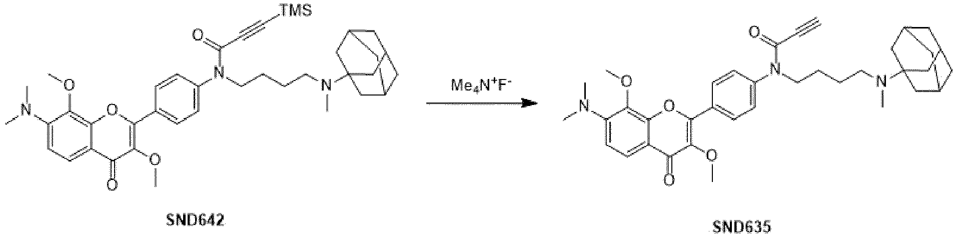

To a solution of **SND642** (180 mg, 263.18 µmol, 1 eq) in DCM (2 mL) was added Me4N⁺F⁻ (24.51 mg, 263.18 µmol, 1 eq), and then stirred at 25 °C for 2h. TLC (SlO₂ , DCM/MeOH= 10/1 , Rf = 0.3) showed **SND642** was consumed and a new spot detected. The reaction mixture was concentrated under reduced pressure to give a residue. The residue was purified by column chromatography (Al₂O₃, petroleum ether/ethyl acetate = 50/1 to 3/1 to 0/1). **SND635** (150 mg, 242.74 µmol, 92.23% yield, 99% purity) was obtained as a yellow solid.
**LCMS:** MS (ESI) Retention time: 0.497 min, (M+1) ⁺ =612.4
**¹H NMR** (400 MHz, CHLOROFORM-d) δ = 8.26-8.24 (br d, J = 8.0 Hz, 2H), 7.87-7.85 (br d, J = 8.8 Hz, 1H), 7.43-7.41 (br d, J = 8.4 Hz, 2H), 6.92-6.90 (br d, J = 9.2 Hz, 1H), 3.95 (s, 3H), 3.90 (s, 3H), 3.88 - 3.81 (m, 2H), 3.05 (s, 6H), 2.86 (s, 1H), 2.37-2.35 (br t, J = 6.8 Hz, 2H), 2.17 (s, 3H), 2.03 (s, 3H), 1.62 - 1.52 (m, 14H), 1.49 - 1.39 (m, 2H).

### A.6 Synthesis of compounds SND644 and SND645

The compounds SND644 and SND645 were synthesized following the reaction sequence depicted in Scheme 4.

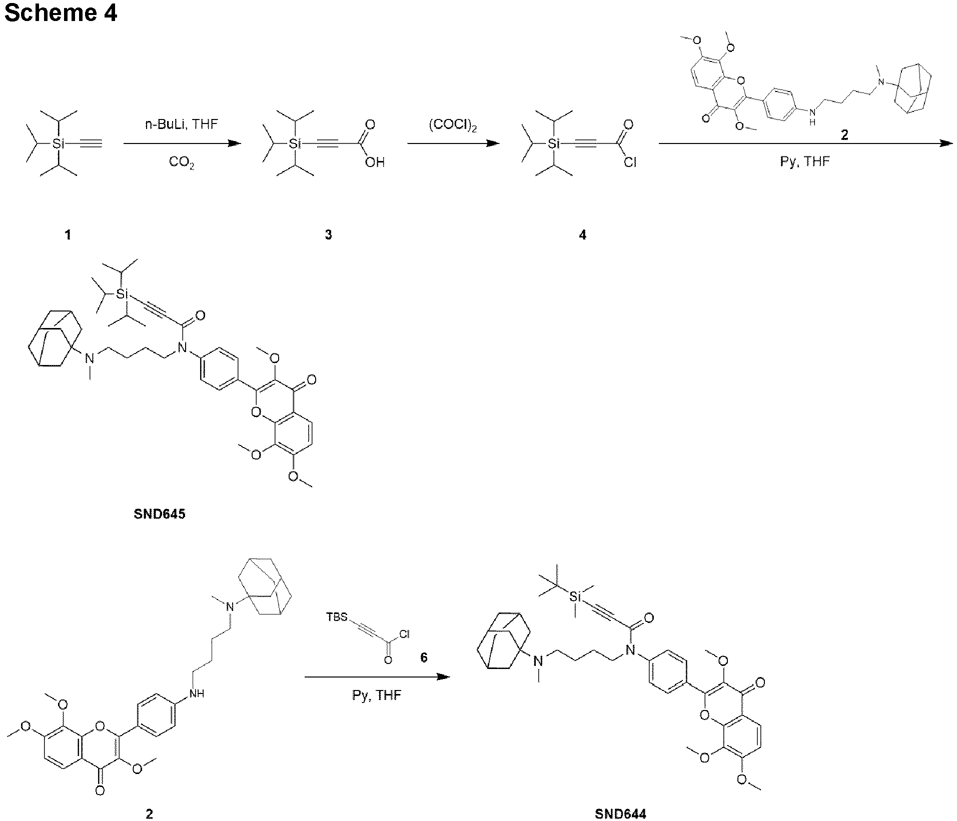

### A.6.1 Synthesis of compound SND644

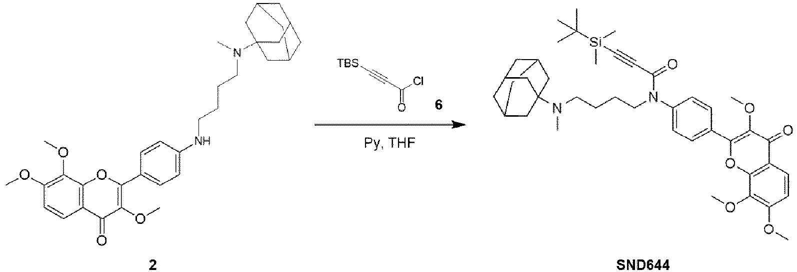

To a solution of **compound** 2 obtained as described at section A.1.1 (50 mg, 91.46 µmol, 1 eq) in THF (2 mL) was added pyridine (21.70 mg, 274.38 µmol, 22.15 µL, 3 eq), the mixture was stirred at 0 °C for 5 min, then the mixture was added **compound 6** (22.25 mg, 109.75 µmol, 1.2 eq) at 0 °C, the mixture was stirred at 0 °C for 2 HR. The mixture was filtered and concentrated in vacuum. The residue was purified by prep_HPLC (column: Phenomenex luna C18 150*25 mm* 10um; mobile phase: [H₂O (0.225% FA) - ACN]; gradient:32%-62% B over 12.0 min). **SND 644** (55.1 mg, 76.35 µmol, 83.48% yield, 98.8% purity) was obtained as yellow gum.
**LCMS:** MS (ESI) Retention time: 0.629 min, (M+1)⁺ =713.3
**¹H NMR** (400 MHz, METHANOL-d₄) δ = 8.26 (d, J = 8.6 Hz, 2H), 7.93 (d, J = 9.0 Hz, 1H), 7.60 (d, J = 8.6 Hz, 2H), 7.28 (d, J = 9.2 Hz, 1H), 4.03 (s, 3H), 3.98 (s, 3H), 3.94 - 3.84 (m, 5H), 2.80 (br s, 2H), 2.48 (s, 3H), 2.15 (br s, 3H), 1.84 (br s, 5H), 1.75 - 1.63 (m, 11H), 0.73 (s, 9H), - 0.01 (s, 6H).

### A.6.2 Synthesis of compound SND645

### A.6.2.1 Synthesis of compound 3

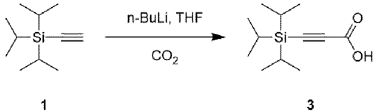

Solution 1: **compound 1** (20 g, 109.66 mmol, 24.60 mL, 1 eq) } in {THF, 400 mL}. Solution 2: n-BuLi (1.6 M, 102.81 mL, 1.5 eq). The solution 1 was pumped by Pump 1 {S₁, P1, 47.206 mL/min} to flow reactor 1 {FLR₁, PFA, Coils reactor, 1.588 (1/16") mm, 5.0 mL, - 40.0 °C}. The solution 2 was pumped by Pump 2 {S₂, P2, 11.618 mL/min} to flow reactor 1 {FLR₁, PFA, Coils reactor, 1.588 (1/16") mm, 5.0 mL, -40.0°C}. The residence time of flow reactor 1 {FLR₁, 0.085 min}. Et₂O (50 mL) and aqueous 10% HCl were added, the organic phase separated, washed with distilled water (3 x 50 mL), dried (MgSO₄), treated with activated charcoal, and the solvent removed in vacuo gave the desired product, which was taken the next step without other purification. 3-triisopropylsilylprop-2-ynoic acid (60 g, crude) was obtained as a colorless oil.
**LCMS:** MS (ESI) Retention time: 0.668 min, (M+1) ⁺ =227.2
**¹H NMR** (400 MHz, CHLOROFORM-d) δ = 10.37 (br s, 1H), 1.08 (s, 3H), 0.98 - 0.86 (m, 18H)

### A.6.2.2 Synthesis of compound 4

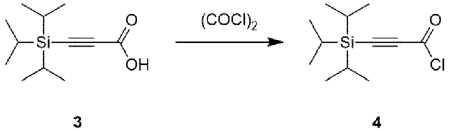

To a solution of **compound 3** (100 mg, 441.72 µmol, 1 eq) was added SOCl₂ (525.52 mg, 4.42 mmol, 320.83 µL, 10 eq), the mixture was stirred at 25 °C for 12 hr. The mixture was filtered and concentrated in vacuum. The residue was taken the next step without other purification. **Compound 4** (108 mg, crude) was obtained as yellow oil.
**LCMS:** MS (ESI) Retention time: 0. 665 min, (M+1) ⁺ =241.3

### A.6.2.3 Synthesis of compound SND645

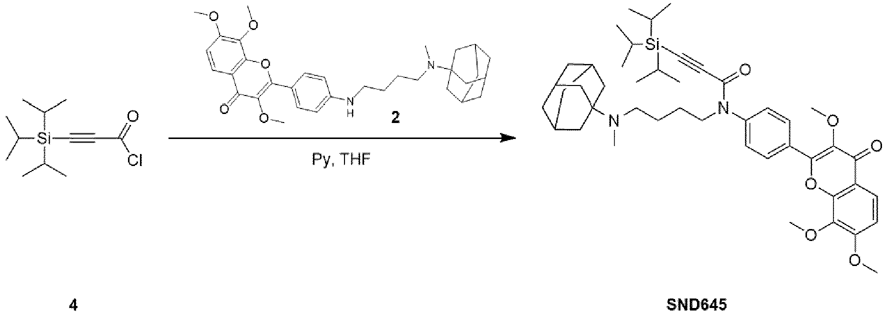

To a solution of **compound 4** (50 mg, 91.46 µmol, 1 eq) in THF (2 mL) was added pyridine (21.70 mg, 274.38 µmol, 22.15 µL, 3 eq), the mixture was stirred at 0 °C for 5 min, then the mixture was added **compound 3** (29.11 mg, 118.90 µmol, 1.3 eq) at 0 °C, the mixture was stirred at 0 °C for 2 HR. The mixture was filtered and concentrated in vacuum. The residue was purified by prep_HPLC. **SND 645** (43.32 mg, 56.74 µmol, 62.04% yield, 98.9% purity) was obtained as yellow gum.
**LCMS:** MS (ESI) Retention time: 0. 673 min, (M+1)⁺ =755.4
**¹H NMR** (400 MHz, METHANOL-d₄) δ = 8.26 (d, *J =* 8.5 Hz, 2H), 7.93 (d, *J* = 9.1 Hz, 1H), 7.61 (d, *J = 8.5* Hz, 2H), 7.29 (d, *J* = 9.1 Hz, 1H), 4.03 (s, 3H), 3.99 (s, 3H), 3.96 - 3.84 (m, 5H), 2.79 (br s, 2H), 2.48 (s, 2H), 2.15 (br s, 3H), 1.96 - 1.46 (m, 17H), 1.35 - 1.12 (m, 3H), 0.92 (s, 18H).

### A.7 Synthesis of compounds SND608 and SND607

The compounds SND608 and SND608TMS were synthesized following the reaction sequence depicted in Scheme 5.

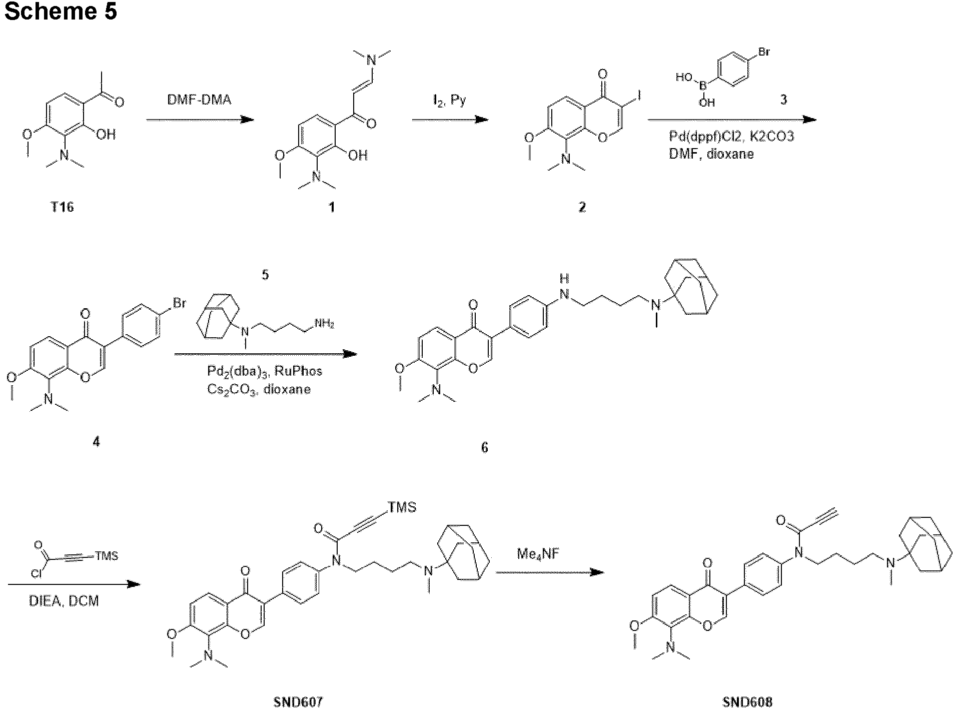

### A.7.1 Synthesis of compound 1

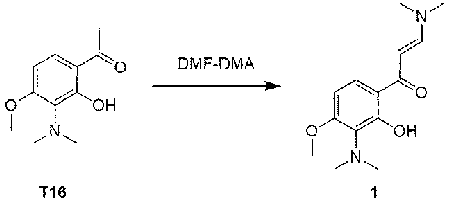

To a solution of **compound T16** (2 g, 9.56 mmol, 1 eq) in MeOH (20 mL) was added DMF-DMA (6.83 g, 57.35 mmol, 7.62 mL, 6 eq), and then the mixture was stirred at 95 °C for 16h under N2. TLC (SiO₂, petroleum ether / ethyl acetate =1/1, Rf = 0.4) showed **compound T16** was consumed and a new spot detected. The reaction mixture was concentrated under reduced pressure to give a residue. The residue was purified by column chromatography (SiO₂, Petroleum ether/Ethyl acetate=50/1 to 1/4). **Compound 1** (3.2 g, crude) was obtained as a yellow solid.
**LCMS:** MS (ESI) Retention time: 0.358 min, (M+1) ⁺ =265.1

### A.7.2 Synthesis of compound 2

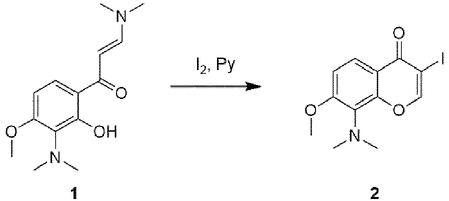

To a solution of **compound 1** (2.5 g, 9.46 mmol, 1 eq) in DCM (20 mL) was added pyridine (2.99 g, 37.83 mmol, 3.05 mL, 4 eq), I2 (2.40 g, 9.46 mmol, 1.91 mL, 1 eq), and then the mixture was stirred at 25 °C for 16h. TLC (SiO2, petroleum ether/ethyl acetate=3/1, Rf =0.4) showed **compound 1** was consumed and two new spots detected. The reaction mixture was added Na₂SO₃ 20 ml, and extracted with DCM (10ml × 2), the organic phase was dried over Na₂SO₄, filtered and concentrated under reduced pressure to give a residue. The residue was purified by column chromatography (SiO₂, Petroleum ether/Ethyl acetate=50/1 to 1/1 to 0/1). **Compound 2** (300 mg, 869.23 µmol, 9.19% yield) was obtained as a light yellow solid.
**LCMS:** MS (ESI) Retention time: 0.363 min, (M+1) ⁺ =346.0

### A.7.3 Synthesis of compound 4

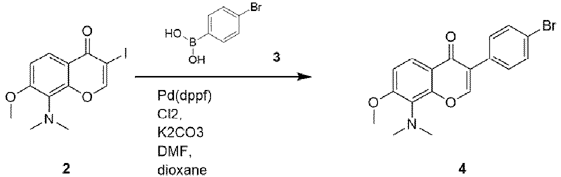

To a mixture of **compound 2** (90 mg, 260.77 µmol, 1 eq), Pd(PPh3)4 (30.13 mg, 26.08 µmol, 0.1 eq), Na2CO3 (2 M, 391.15 µL, 3 eq) in toluene (1 mL) was added a solution of compound 3 (78.55 mg, 391.15 µmol, 1.5 eq) in EtOH (1 mL) , and then the mixture was sitrred at 50 °C for 3h under N2. TLC (SiO₂, petroleum ether / ethyl acetate =3/1, Rf= 0.5) showed compound 2 was consumed and a new spot detected. The reaction mixture was extracted with ethyl acetate (10 ml × 2) and H2O (20 ml), the organic phase was dried over Na2SO4, filtered and concentrated under reduced pressure to give a residue. The residue was purified by column chromatography (SiO₂, Petroleum ether/Ethyl acetate=50/1 to 3/1 to 0/1). **Compound 4** (230 mg, 590.01 µmol, 75.42% yield, 96% purity) was obtained as a yellow solid.
**LCMS:** MS (ESI) Retention time: 0.544 min, (M+1) ⁺ =374.1

### A.7.4 Synthesis of compound 6

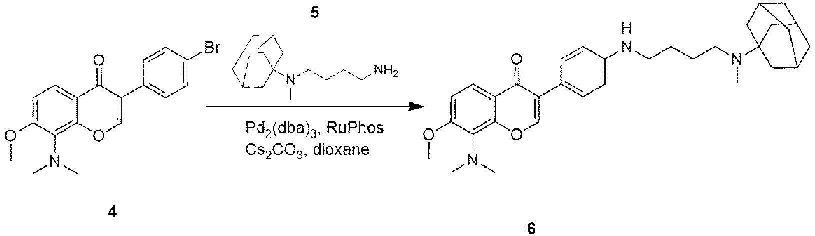

A suspension of **compound 4** (200 mg, 534.43 µmol, 1 eq), **compound 5** (151.61 mg, 641.32 µmol, 1.2 eq), Gphos-Pd-G6 (50.47 mg, 53.44 µmol, 0.1 eq), t-BuONa (1 M, 400.83 µL, 1.5 eq) in dry dioxane (2 mL) was stirred at 65 °C under N2 for 16h. The reaction mixture was filtered and then concentrated under reduced pressure to give a residue. The crude product was purified by reversed-phase HPLC (0.1 % FA condition). **Compound 6** (220 mg, 353.02 µmol, 66.06% yield, 85% purity) was obtained as a yellow oil.
**LCMS:** MS (ESI) Retention time: 0.537 min, (M+1) ⁺ =530.2

### A.7.5 Synthesis of compound SND607

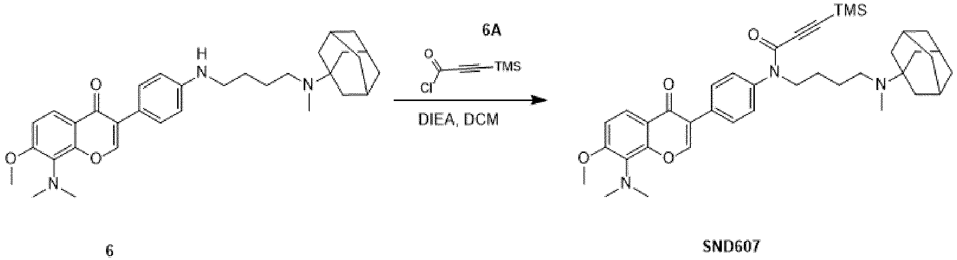

To a solution of **compound 6** (120 mg, 226.54 µmol, 1 eq), pyridine (53.76 mg, 679.62 µmol, 54.85 µL, 3 eq) in THF (1.5 mL) was added compound 6A (54.60 mg, 339.81 µmol, 1.5 eq) at 0 °C , and then stirred at 0 °C for 10 min. It was used into the next step directly. **SND607** (150 mg, crude) was obtained as a yellow oil.
**LCMS:** MS (ESI) Retention time: 0.474 min, (M+1) ⁺ =654.6

### A.7.6 Synthesis of compound SND608

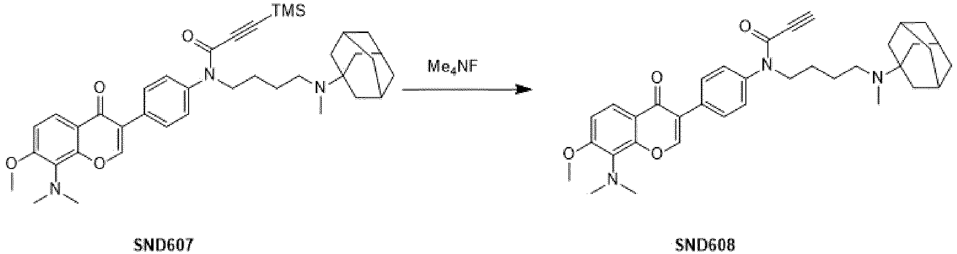

To a solution of **SND608** (150 mg, 229.38 µmol, 1 eq) in THF (1.5 mL) was added Me₄NF (17.09 mg, 183.51 µmol, 0.8 eq), and then the mixture was stirred at 25 °C for 2h. TLC (SiO₂ , DCM / MeOH = 10/1, Rf = 0.4) showed **compound 7** was consumed and a new spot detected. The reaction mixture was concentrated under reduced pressure to give a residue. The residue was purified by column chromatography (Al₂O₃, Petroleum ether/Ethyl acetate=50/1 to 1/1 to 0/1). **SND608** (95 mg, 156.02 µmol, 68.02% yield, 95.54% purity) was obtained as a yellow oil.
**LCMS:** MS (ESI) Retention time: 0.422 min, (M+1)⁺ =582.4, EW53323-445-P1A
**¹H NMR** (400 MHz, CHLOROFORM-d) δ = 8.12 - 8.03 (m, 2H), 7.63 (br d, J = 7.6 Hz, 2H), 7.34 - 7.28 (m, 2H), 7.03 (br d, J = 8.8 Hz, 1H), 3.97 (s, 3H), 3.77 (br t, J = 6.8 Hz, 2H), 2.92 (s, 6H), 2.85 (s, 1H), 2.40 (br s, 2H), 2.20 (s, 3H), 2.04 (br s, 3H), 1.65 (br s, 7H), 1.60 - 1.52 (m, 7H), 1.50 - 1.43 (m, 2H)

### A.8 Synthesis of compounds SND610 and SND611

The compounds SND610 and SND611 were synthesized following the reaction sequence depicted in Scheme 6.

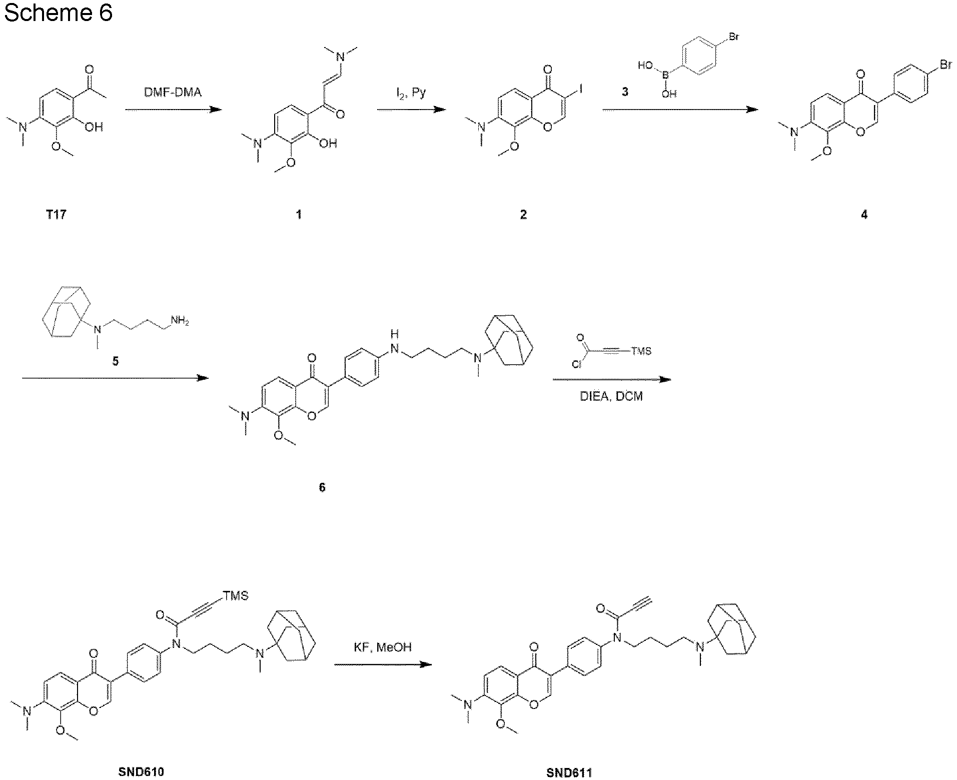

### A.8.1 Synthesis of compound 1

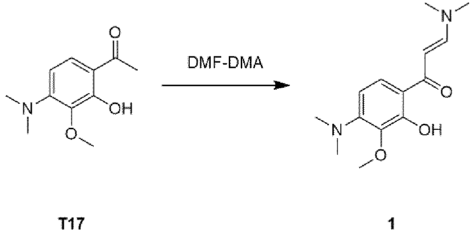

To a solution of **Compound T17** (1.0 g, 4.78 mmol, 1 eq) in MeOH (10 mL) was added DMF-DMA (3.42 g, 28.68 mmol, 3.81 mL, 6 eq) at 25 °C, the mixture was stirred at 100 °C for 12 hr. TLC (petroleum ether/ethyl acetate = 1:1, Rf = 0.35) showed the **Compound T17** was consumed. The mixture was filtered and concentrated in vacuum. The residue was purified by silica gel chromatography (column height: 250 mm, diameter: 100 mm, 100-200 mesh silica gel, Petroleum ether/Ethyl acetate = 20/1, 1/1). **Compound 1** (0.9 g, 3.10 mmol, 64.83% yield, 91% purity) was obtained as yellow oil.
**LCMS:** MS (ESI) Retention time: 0.394 min, (M+1) ⁺ =265.1

### A.8.2 Synthesis of compound 2

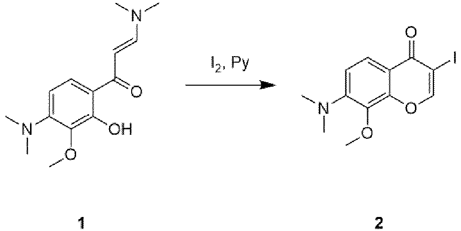

To a solution of **compound 1** (0.9 g, 3.40 mmol, 1 eq) in CHCl₃ (9 mL) was added pyridine (1.08 g, 13.62 mmol, 1.10 mL, 4 eq) and I₂ (950.63 mg, 3.75 mmol, 754.47 µL, 1.1 eq), the mixture was stirred at 25 °C for 12 hr. TLC (petroleum ether/ethyl acetate = 5:1, Rf = 0.45) showed the compound 1 was consumed. The mixture was added aq. Na₂SO₃ and DCM (10 mL), the aqueous phase was extracted with DCM (5 mL × 3). The combined organic phase was washed with brine (15 mL), dried with anhydrous Na₂SO₄, filtered and concentrated in vacuum. The residue was purified by silica gel chromatography (column height: 250 mm, diameter: 100 mm, 100-200 mesh silica gel, Petroleum ether/Ethyl acetate = 20/1, 1/1). 7-(dimethylamino) -3-iodo-8-methoxy-chromen-4-one (760 mg, 2.20 mmol, 64.67% yield, 100% purity) was obtained as white solid.
**¹H NMR** (400 MHz, CHLOROFORM-d) δ = 8.24 (s, 1H), 7.86 (d, J = 9.0 Hz, 1H), 6.91 (d, J = 9.0 Hz, 1H), 3.83 (s, 3H), 3.06 (s, 6H)

### A.8.3 Synthesis of compound 4

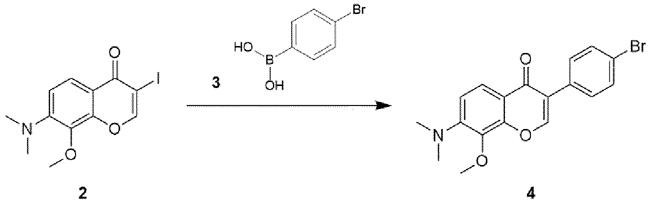

To a solution of compound 2 (300 mg, 869.23 µmol, 1 ***eq***), Pd(PPh₃)₄ (100.45 mg, 86.92 µmol, 0.1 ***eq***) and Na₂CO₃ (276.39 mg, 2.61 mmol, 3 ***eq***) in toluene (3 mL) was added a solution of compound 3 (209.48 mg, 1.04 mmol, 1.2 ***eq***) in EtOH (3 mL), and the mixture was stirred at 50 °C for 12 hr under N₂. The residue was poured into water (10 mL) and stirred for 5 min. The aqueous phase was extracted with DCM (10 mL × 3). The combined organic phase was washed with brine (15 mL), dried with anhydrous Na₂SO₄, filtered and concentrated in vacuum. The residue was purified by silica gel chromatography (column height: 250 mm, diameter: 100 mm, 100-200 mesh silica gel, Petroleum ether/Ethyl acetate = 50/1, 3/1). The residue was purified by prep_ HPLC (column: Phenomenex luna C18, 150 × 25 mm × 10 um; mobile phase: [water(FA)-ACN]; gradient:53%-83% B over 10 min). **Compound 4** (140 mg, 374.10 µmol, 43.04% yield, 100% purity) was obtained as white solid.
**LCMS:** MS (ESI) Retention time: 0.678 min, (M+1) ⁺ =374.1
**¹H NMR** (400 MHz, DMSO-d₆) δ = 8.50 (s, 1H), 7.73 (d, *J* = 9.0 Hz, 1H), 7.65 - 7.60 (m, 2H), 7.58 - 7.52 (m, 2H), 7.03 (d, *J* = 9.2 Hz, 1H), 3.80 (s, 3H), 3.02 (s, 6H)

### A.8.4 Synthesis of compound 6

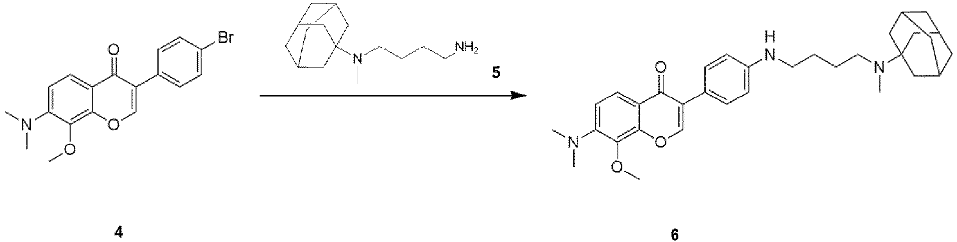

To a solution of **Compound 4** (150 mg, 400.83 µmol, 1 eq), **Compound 5** (132.65 mg, 561.16 µmol, 1.4 eq), bromo-[4-(2-trimethylsilylethoxycarbonyl) phenyl]palladium;[6-tert-butoxy-2-(2, 6-diisopropylphenyl) -3-methoxy-phenyl]-dicyclohexyl-phosphane (37.86 mg, 40.08 µmol, 0.1 eq) and t-BuONa (2 M, 300.62 µL, 1.5 ***eq***) in dioxane (2 mL) at 25 °C, the mixture was stirred at 65 °C for 3 hr under N₂. The residue was poured into water (5 mL) and stirred for 2 min. The aqueous phase was extracted with ethyl acetate (10 mL × 3). The combined organic phase was washed with brine (15 mL), dried with anhydrous Na₂SO₄, filtered and concentrated in vacuum. The residue was purified by perp_ HPLC (FA). **Compound 6** (0.17 g, 320.93 µmol, 80.07% yield, 100% purity) was obtained as yellow oil.
**LCMS:** MS (ESI) Retention time: 0.501 min, (M+1)⁺ =530.4

### A.8.5 Synthesis of SND610

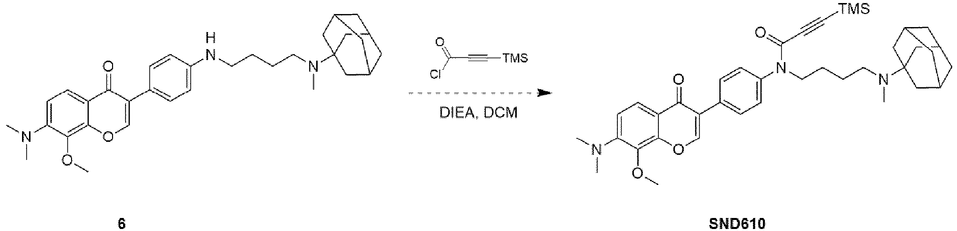

To a solution of **Compound 6** (120 mg, 226.54 µmol, 1 ***eq***) in THF (1 mL) was added pyridine (53.76 mg, 679.62 µmol, 54.85 µL, 3 eq), the mixture was stirred at 0 °C for 5 min, then the mixture was added₃-trimethylsilylprop-2-ynoyl chloride (54.60 mg, 339.81 µmol, 1.5 eq) at 0 °C, the mixture was stirred at 0 °C for 2 hr. The mixture was concentrated in reduced pressure at 25 °C. The residue was poured into water (10 mL). The aqueous phase was extracted with DCM (10 mL × 2). The combined organic phase was poured into aq. Citric acid (10 mL), the aqueous phase was extracted with DCM (10 mL × 2). The combined organic phase was poured into aq. NaHCO₃ (10 mL), the aqueous phase was extracted with DCM (10 mL × 2), The combined organic phase was poured into water (10 mL). The aqueous phase was extracted with DCM (10 mL × 2) dried with anhydrous Na₂SO₄, filtered and concentrated in vacuum. The residue was purified by prep-HPLC (column: Phenomenex luna C18, 150 × 25 mm × 10 um; mobile phase: [water(FA)-ACN];gradient:28%-58% B over 10 min). **SND610** (180 mg, crude) was obtained as white solid.
**LCMS:** MS (ESI) Retention time: 0.571 min, (M+1) ⁺ =654.6

### A.8.6 General procedure for preparation of SND611

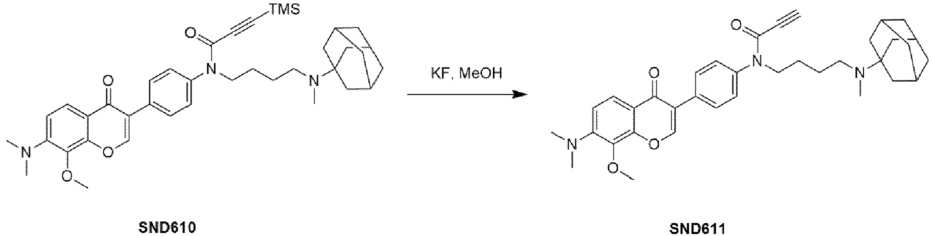

To a solution of **SND610** (121 mg, 185.04 µmol, 1 eq) in THF (1.5 mL) was added KF (13.79 mg, 148.03 µmol, 0.8 eq), the mixture was stirred at 25 °C for 12 hr. The residue was poured into water (10 mL). The aqueous phase was extracted with ethyl acetate (5 mL × 3). The combined organic phase was washed with brine (15 mL), dried with anhydrous Na₂SO₄, filtered and concentrated in vacuum. The residue was taken the next step without other purification. **SND611** (69.26 mg, 113.10 µmol, 61.12% yield, 95% purity) was obtained as black brown solid.
**LCMS:** MS (ESI) Retention time: 0.566 min, (M+1) ⁺ =582.4
**¹H NMR** (400 MHz, CHLOROFORM-d) δ = 8.07 (s, 1H), 7.93 (d, *J* = 9.0 Hz, 1H), 7.67 (d, *J =* 8.3 Hz, 2H), 7.33 (d, *J* = 8.3 Hz, 2H), 6.95 (d, *J* = 9.0 Hz, 1H), 3.89 (s, 3H), 3.86 - 3.72 (m, 1H), 3.07 (s, 6H), 2.91 - 2.84 (m, 1H), 2.67 (s, 3H), 2.29 - 2.17 (m, 4H), 2.06 (br s, 7H), 1.78 (br d, *J* = 10.5 Hz, 6H), 1.68 - 1.52 (m, 5H).

### A.9 Synthesis of compounds SND632 and SND633

The compounds SND632 and SND633 were synthesized following the reaction sequence depicted in Scheme 7.

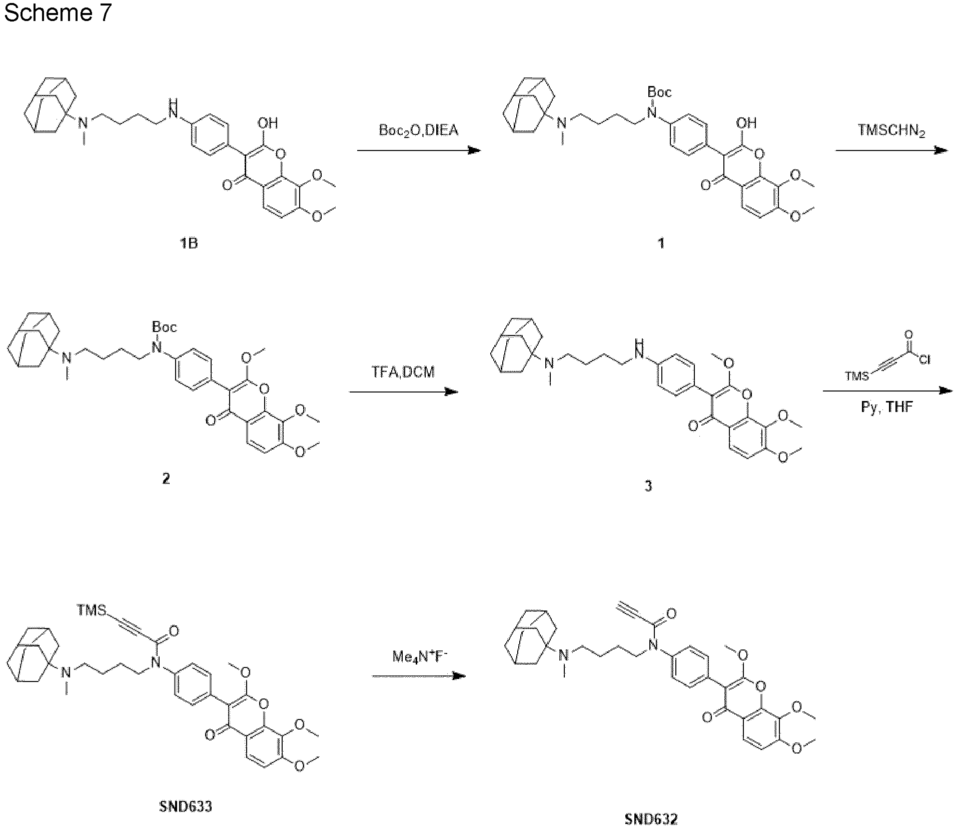

### A.9.1 Synthesis of compound 1

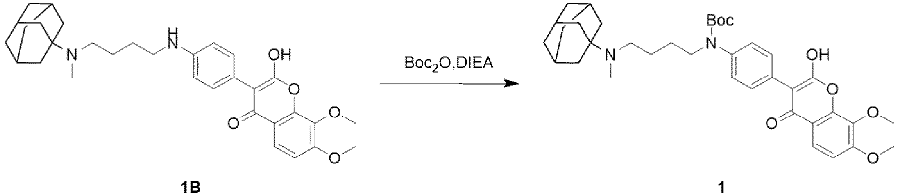

To a solution of **compound 1B** (400 mg, 750.94 µmol, 1 ***eq***) in DCM (4 mL) was added DIEA (194.11 mg, 1.50 mmol, 261.60 µL, 2 ***eq***) and Boc₂O (196.67 mg, 901.12 µmol, 207.02 µL, 1.2 ***eq***), the mixture was stirred at 30 °C for 12 hr. The mixture was filtered and concentrated in vacuum. The mixture was taken the next step without other purification. **Compound 1** (470 mg, 742.75 µmol, 98.91% yield) was obtained as yellow solid.
**LCMS:** MS (ESI) Retention time: 0.609 min, (M+1)⁺ =633.3

### A.9.2 Synthesis of compound 2

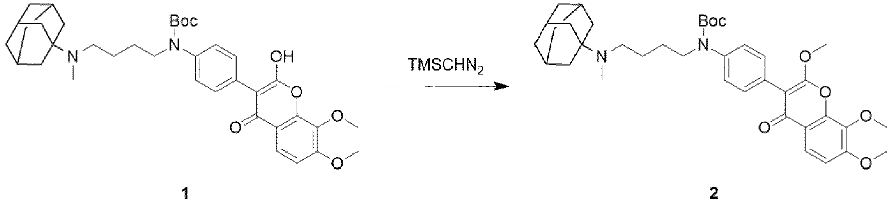

To a solution of **compound 1** (470 mg, 742.75 µmol, 1 eq) in DCM (5 mL) was added TMSCHN₂ (509.03 mg, 4.46 mmol, 6 eq), the mixture was stirred at 25 °C for 12 hr. The mixture was added AcOH (5 mL), and the mixture was stirred at 25 °C for 30 min, the mixture filtered and concentrated in vacuum. The mixture was taken the next step without other purification. **Compound 2** (470 mg, 726.64 µmol, 97.83% yield) was obtained as yellow oil.
**LCMS:** MS (ESI) Retention time: 0.599 min, (M+1) ⁺ =647.4

### A.9.3 Synthesis of compound 3

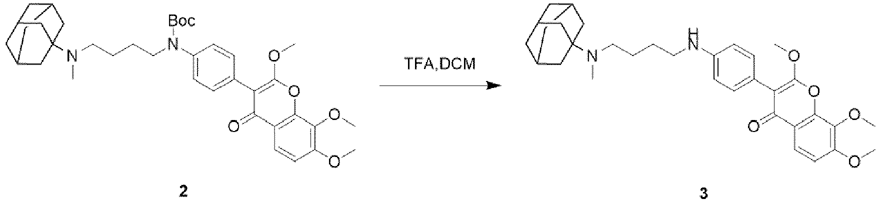

To a solution of **compound 2** (470 mg, 726.64 µmol, 1 eq) in TFA (3 mL), the mixture was stirred at 25 °C for 3 min. The mixture was filtered and concentrated in vacuum. The residue was purified by prep_ HPLC (column: Phenomenex Luna C18, 150 × 25 mm × 10 um; mobile phase: [H₂O (0.225% FA)-ACN]; gradient: 15%-45% B over 15.0 min). **Compound 3** (100 mg, 182.92 µmol, 25.17% yield) was obtained as yellow oil.
**LCMS:** MS (ESI) Retention time: 0.475 min, (M+1) ⁺ =547.4
**¹H NMR** (400 MHz, DMSO-d₆) δ = 7.54 (d, *J* = 8.9 Hz, 1H), 7.12 (d, J = 8.8 Hz, 3H), 6.62 (d, J = 8.6 Hz, 2H), 5.87 (br d, J = 2.8 Hz, 1H), 3.92 (s, 3H), 3.83 (s, 3H), 3.52 (s, 3H), 3.16 - 3.02 (m, 3H), 2.98 - 2.74 (m, 2H), 2.12 (br s, 3H), 1.81 (br s, 6H), 1.62 (br s, 10H).

### A.9.4 Synthesis of compound SND633

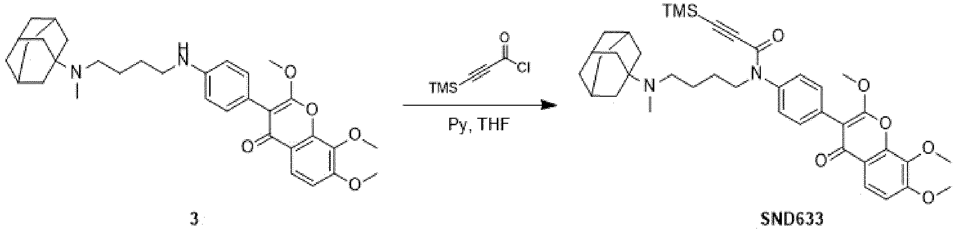

To a solution of **Compound** 3 (50 mg, 91.46 µmol, 1 ***eq***) in THF (2 mL) was added pyridine (21.70 mg, 274.38 µmol, 22.15 µL, 3 ***eq***), the mixture was stirred at 0 °C for 5 min, then the mixture was added 3-trimethylsilylprop-2-ynoyl chloride (17.63 mg, 109.75 µmol, 1.2 ***eq***) at 0 °C, the mixture was stirred at 0 °C for 5 min. The mixture was filtered and concentrated in vacuum. The residue was taken to the next step without other purification. **SND633** (80 mg, crude) was obtained as yellow oil.
**LCMS:** MS (ESI) Retention time: 0.572 min, (M+1) ⁺ = 671.5

### A.9.5 Synthesis of compound SND632

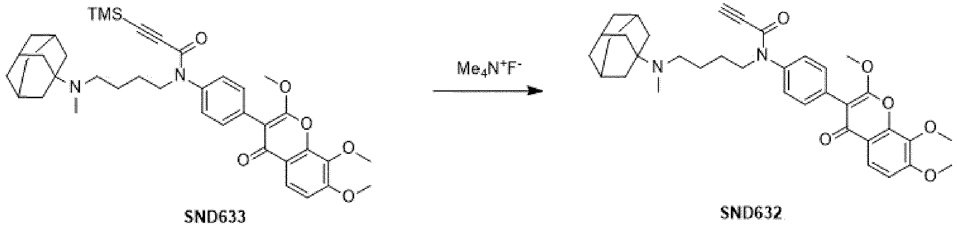

To a solution of **SND633** (80 mg, 119.24 µmol, 1 ***eq***) in THF (1 mL) was added tetramethylammonium fluoride (33.32 mg, 357.72 µmol, 3 ***eq***), the mixture was stirred at 25 °C for 2 hr. TLC (DCM : MEOH = 10 :1) showed the compound 4 was consumed and desired mass was detected. The mixture was filtered and concentrated in vacuum. The residue was purified by silica gel chromatography (column height: 250 mm, diameter: 100 mm, 100-200 mesh Al₂O₃, Petroleum ether/Ethyl acetate = 10/1, 0/1). **SND632** (20 mg, 31.07 µmol, 26.05% yield, 93% purity) was obtained as yellow solid.
LCMS: MS (ESI) Retention time: 0.510 min, (M+1)⁺ =599.5

### B. Biological studies

### B.1 Evaluation of anti-proliferative effect of the compounds in a panel of cancer cell lines

The antitumor activity of the compounds according to the present invention and SND462 as comparative example in different cell lines was assessed by using ATPLite 1Step chemiluminescence assay (Revvity, Groningen, the Netherlands) according to the manufacturer's protocol. ATPLite 1Step chemiluminescence assay relies upon the use of intracellular ATP content as indirect readout of cell number.

To ensure unrestricted growth and maximal signal at the end of the experiment, cells were seeded at an optimized density in 384-well plates. The starting cell number was determined by adding ATPLite to each well of a control plate after 24 hours of incubation and recording luminescence on an Envision multimode reader (Revvity, Waltham, MA) (Uitdehaag 2014).

A 9-point dilution series for each tested compound was obtained by diluting the tested compound in DMSO with √10-fold steps from a 10 mmol/L stock and a 31.6-fold dilution in 20 mmol/L HEPES (pH7.4). Final DMSO concentration in all wells was 0.4% (v/v). The dilution series was added in duplicate to the cells in the 384-well plates to determine the effect of the tested compound on cell viability. Vehicle-treated controls were included to determine maximal cell growth. Intracellular ATP content was measured in each well using ATPLite after 72 hours of incubation with compound. The luminescent signal was normalized with vehicle-treated control wells. Cell doublings were calculated by dividing the vehicle-treated control cell number by the starting cell number for each cell line. Assay reproducibility was evaluated by a parallel test with doxorubicin and monitoring the cell doubling of each cell line. A four-parameter logistic model was fitted to the percentage viability values using IDBS XLfit5 (IDBS, Guildford, United Kingdom) to calculate the maximum inhibitory concentration (IC₅₀) values. IC₅₀ values of invalidated curves (F-value > 1.5) were maximized at the highest concentration.

**Table 1: Tumor cell lines and designation.**

| **Cancer Origin** | **Cancer type** | **Cell line** |
|---|---|---|
| Brain | Neuroblastoma | Kelly |
| | Desmoplastic Cerebellar Medulloblastoma | Daoy |
| | Neuroblastoma | IMR-32 |
| | Glioblastoma | LN-229 |
| | Neuroblastoma | SK-N-AS |
| | Glioblastoma | U118 MG |
| | Glioblastoma | U87 MG |
| Skin | Melanoma | A-2058 |
| | Melanoma | G-361 |
| | Melanoma | RPMI-7951 |
| | Melanoma | SK-MEL-2^{a)} |
| | Melanoma | SK-MEL-5 |
| | Melanoma | SK-MEL-28 |
| | Melanoma | A-375 |
| Bladder | Carcinoma | 5637 |
| | Carcinoma | J82 |
| | Carcinoma | RT112/84 |
| | Carcinoma | RT-4 |
| | Carcinoma | SW780 |
| | Carcinoma | T24 |
| Blood-leukemia | Chronic myeloid leukemia (CML) | K-562 |
| | T-cell acute lymphoblastic leukemia (ALL) | CCRF-CEM |
| | Chronic eosinophilic | EOL-1 |
| | Acute myeloid leukemia (AML) | HL-60 |
| | Acute lymphoblastic leukemia (ALL) | Jurkat E6.1 |
| | B-cell acute lymphoblastic leukemia (ALL) | JVM-3 |
| | Acute myeloid leukemia | KG-1 |
| | Chronic myeloid BCRABL1 positive leukemia (CML) | Ku812 |
| | Acute myeloid leukemia (AML) | ML-2 |
| | Acute myeloid leukemia (AML) | MOLM-13 |
| | Acute myeloid leukemia (AML) | MOLT-4 |
| | Acute lymphoblastic leukemia (ALL) | NALM-6 |
| | Acute myeloid leukemia (AML) | OCI-AML3 |
| | Acute lymphoblastic leukemia (ALL) | RS4;11 |
| | T-cell acute lymphoblastic leukemia (ALL) | PEER |
| Blood lymphoma | Burkitt's lymphoma | Daudi |
| | Diffuse large B-cell lymphoma | DB |
| | B-cell lymphoma | GRANTA-519 |
| | Diffuse large B-cell | HT |
| | Cutaneous T-cell lymphoma | HuT 102 |
| | Cutaneous T-cell lymphoma (Sezary syndrom) | HuT 78 |
| | Mantel cell lymphoma | JEKO1 |
| | Anaplastic large cell lymphoma | KARPAS 299 |
| | Germinal center B-cell like diffuse large B-cell lymphoma | KARPAS 422 |
| | ALK-positive anaplastic large cell lymphoma | L-82 |
| | Burkitt's lymphoma | NAWALMA |
| | B-cell lymphoma | OCI-LY19 |
| | Diffuse large B-cell lymphpma | OCI-LY7 |
| | Diffuse large B-cell lymphoma | OCI-LY3 |
| | Diffuse Large B-Cell Lymphoma GCB | Pfeiffer |
| | Mantle cell lymphoma | REC1 |
| | Burkitt's lymphoma | Raji |
| | Burkitt's lymphoma | Ramos |
| | B cell lymphoma | RL |
| | ALK-positive anaplastic large cell lymphoma | SR |
| | ALK-positive anaplastic large cell | SU-DHL-1 |
| | Diffuse large B-cell lymphoma | SU-DHL-10 |
| | Large cell lymphoma | SU-DHL-2 |
| | Diffuse Large B-Cell Lymphoma GCB | SU-DHL-4 |
| | Large cell lymphoma | SU-DHL-8 |
| | Diffuse Large B-Cell Lymphoma | Toledo |
| | Histiocytic | U937 |
| | Burkitt's lymphoma | CA46 |
| | B-lymphoblastoid | ARH-77 |
| Blood myeloma | Plasma cell myeloma | JJN-3 |
| | Plasma cell myeloma | KMS-11 |
| | Plasma cell myeloma | L-363 |
| | Multiple Myeloma | MM.1R^{a)} |
| | Plasma cell myeloma | MM.1S |
| | Plasma cell myeloma | MOLP8 |
| | Plasmacytoma | NCI-H929 |
| | Plasma cell myeloma | OPM2 |
| | Plasmacytoma | RPMI-8226 |
| | Multiple Myeloma | U266B1 |
| | Plasma cell myeloma | ALMC-2 |
| | Plasma cell myeloma | ALMC-1 |
| | Plasma cell myeloma | AMO-1 |
| Head and Neck | Tongue Squamous Cell Carcinoma | CAL-27 |
| | Pharynx Squamous Cell Carcinoma | Detroit 562 |
| | Pharynx Squamous Cell Carcinoma | FaDu |
| | Tongue Squamous Cell Carcinoma | SCC-4 |
| Kidney | Clear cell carcinoma | Caki-1 |
| | Adrenal gland carcinoma | NCI-H295R |
| | Papillary carcinoma | ACHN |
| | Adenocarcinoma | 786-O |
| | Carcinoma | A-498 |
| Intestines | Duodenal adenocarcinoma | HuTu 80 |
| | Colorectal Adenocarcinoma (Duke's type D) | COLO 205 |
| | Colorectal Adenocarcinoma (Duke's type C) | DLD-1 |
| | Colorectal Adenocarcinoma | HT-29 |
| | Colorectal Adenocarcinoma | HT-55 |
| | Colorectal Carcinoma | HCT 116 |
| | Colorectal Adenocarcinoma (Duke's type C) | LoVo^{a)} |
| | Cecum adenocarcinoma | LS1034 |
| | Colorectal Adenocarcinoma (Duke's type B) | LS174T |
| | Cecum Adenocarcinoma | LS411N |
| | Cecum adenocarcinoma | NCI-H716 |
| | Colon carcinoma | RKO |
| | Cecum Adenocarcinoma | SNU-C2B |
| | Colon Adenocarcinoma | SNU-81 |
| | Colorectal Adenocarcinoma (Dukes type C) | SW1463 |
| | Colorectal Adenocarcinoma (Dukes type C) | SW403 |
| | Rectal Adenocarcinoma | SW837 |
| | Colon Carcinoma (isolated from ovaries) | SW626 |
| | Colorectal Adenocarcinoma (Dukes type C) | SW948 |
| | Colon Adenocarcinoma | CCK-81 |
| Liver | Hepatocarcinoma | Hep G2/C3A |
| | Hepatocarcinoma | Hep3B |
| | Hepatocarcinoma | HepG2 |
| | Hepatocarcinoma | HUH-7 |
| | Hepatocarcinoma | JHH-5 |
| | Hepatocarcinoma | JHH-7 |
| | Hepatocarcinoma | PLC/PRF/5 |
| | Hepatocarcinoma | SNU-878 |
| Lung | Non-small cell lung cancer (NSCLC) | COR-L23 |
| | Small cell lung carcinoma | DMS-114 |
| | Small cell carcinoma | DMS-53 |
| | Squamous Cell Carcinoma | HCC 95 |
| | Squamous Cell Carcinoma | EBC-1 |
| | Non-small cell adenocarcinoma | HCC 4006 |
| | Non-small cell adenocarcinoma | HCC 515 |
| | Non-small cell adenocarcinoma | HCC 827 |
| | Non-small cell adenocarcinoma | HCC 2935 |
| | Non-small cell adenocarcinoma | LC-2/AD |
| | Large cell carcinoma | LCLC-97TM1 |
| | NSCLC | NCI-H1299 |
| | Non-small cell adenocarcinoma | NCI-H1373 |
| | Non-small cell adenocarcinoma | NCI-H1395 |
| | Non-small cell adenocarcinoma | NCI-H1563 |
| | Non-small cell adenocarcinoma | NCI-H1573 |
| | NSCLC | NCI-H1581 |
| | Bronchoalveolar carcinoma | NCI-H1650 |
| | NSCLC | NCI-H1703 |
| | Small cell lung carcinoma | NCI-H1688 |
| | Bronchoalveolar carcinoma | NCI-H1666 |
| | Bronchoalveolar carcinoma | NCI-H1781 |
| | Small cell lung carcinoma | NCI-H187 |
| | Non-small cell adenocarcinoma | NCI-H1975^{a)} |
| | Non-small cell adenocarcinoma, stage 4 | NCI-H1792 |
| | NSCLC | NCI-H2030 |
| | NSCLC | NCI-H1993 |
| | NSCLC | NCI-H2023 |
| | Pleural mesothelioma | NCI-H2052 |
| | NSCLC | NCI-H2110 |
| | Small-cell lung carcinoma | NCI-H209 |
| | NSCLC | NCI-H23 |
| | NSCLC | NCI-H2228 |
| | Small cell Carcinoma | NCI-H292 |
| | Non-small cell adenocarcinoma | NCI-H441 |
| | Small cell lung carcinoma | NCI-H446 |
| | Squamous Cell Carcinoma | NCI-H520 |
| | NSCLC | NCI-H460 |
| | NSCLC | NCI-H661 |
| | NSCLC | NCI-H526 |
| | Small cell lung carcinoma | NCI-H82 |
| | Papillary adenocarcinoma | NCI-H820 |
| | Carcinoid | NCI-H727 |
| | Non-small cell adenocarcinoma | NCI-H838 |
| | Squamous cell carcinoma | SW900 |
| | Non-small cell adenocarcinoma | CALU-6 |
| | Epidermoid Carcinoma (Squamos cell carcinoma) | CALU-1 |
| | Squamous cell carcinoma | A-549 |
| | Non-small cell adenocarcinoma | A-427 |
| | Non-small cell adenocarcinoma | PC-9 |
| | Non-small cell adenocarcinoma | CALU-3 |
| Gynecologic | Endometrial adenocarcinoma | AN3-CA |
| | Endometrial adenocarcinoma | Ishikawa |
| | Endometrial adenocarcinoma | KLE |
| | Ovarian carcinoma | COV504 |
| | Ovarian clear cell adenocarcinoma | ES-2 |
| | Cervical adenocarcinoma | HeLa |
| | Ovarian endometrioid adenocarcinoma | IGR-OV1 |
| | Uterine choriocarcinoma | JAR |
| | Uterine choriocarcinoma | JEG-3 |
| | Ovarian adenocarcinoma | OVCAR-3^{a)} |
| | High grade ovarian serous adenocarcinoma | OVCAR-4 |
| | Ovarian serous adenocarcinoma | OVCAR-5 |
| | High grade ovarian serous adenocarcinoma | OVCAR-8 |
| | Ovarian teratocarcinoma | PA-1 |
| | Ovarian serous cystadenocarcinoma | SK-OV-3^{a)} |
| | Ovarian primary malignant adenocarcinoma | TOV-21G |
| | Ovarian adenocarcinoma | A2780 |
| | Ovarian adenocarcinoma | A2780cis^{a)} |
| | Ovarian clear cell adenocarcinoma | RMG-1 |
| Pancreas | Ductal adenocarcinoma | AsPC-1 |
| | Ductal adenocarcinoma | BxPC-3 |
| | Adenocarcinoma | HPAF-II |
| | Adenocarcinoma | Hs 766T |
| | Adenocarcinoma | HuP-T4 |
| | Ductal adenocarcinoma | Mia-PaCa-2 |
| | Adenocarcinoma | Panc 05.04 |
| | Adenocarcinoma | Panc 03.27 |
| | Ductal adenocarcinoma | Panc-1^{a)} |
| | Adenocarcinoma | PSN-1 |
| | Adenocarcinoma | SW1990 |
| | Ductal adenocarcinoma | Capan-2 |
| | Adenocarcinoma | HuP-74 |
| | Adenocarcinoma | Panc 02.03 |
| | Adenocarcinoma | Hs 766T |
| Prostate | Carcinoma | DU145^{a)} |
| | Carcinoma | NCI-H660 |
| | Carcinoma | PC-3^{a)} |
| | Carcinoma | 22Rv1 |
| Stomach | Carcinoma | HGC-27 |
| | Adenocarcinoma | Hs 746.T |
| | Cholangiocellular carcinoma | HuCCT1 |
| | Adenosquamous carcinoma | MKN1 |
| | Adenocarcinoma | SNU-620 |
| | Adenocarcinoma | SNU-16 |
| | Signet ring cell adenocarcinoma | SNU-601 |
| | Signet ring cell adenocarcinoma | YCC-2 |
| | Adenocarcinoma | YCC-10 |
| Breast | Invasive Carcinoma | Hs 578T |
| | Ductal carcinoma | BT-549 |
| | Ductal carcinoma | HCC 1187 |
| | Ductal carcinoma, grade 3 | HCC 1954^{a)} |
| | Carcinoma | DU4475 |
| | Squamous Cell Carcinoma | HCC 1806^{a)} |
| | Metastatic adenocarcinoma | MDA-MB-231^{a)} |
| | Metastatic adenocarcinoma | MDA-MB-468 |
| | Pleomorphic Carcinoma | SUM 159PT |
| | Ductal carcinoma | T47D |
| | Carcinoma | CAL-51 |
| Soft tissue sarcoma (muscle) | Fibrosarcoma | HT-1080 |
| | Embryonal rhabdomyosarcoma | RD |
| | Embryonal rhabdomyosarcoma | Rh30 |
| | Rhabdomyosarcoma | SJCRH30 |
| | Liposarcoma | SW872 |
| | Biphasic synovial sarcoma | SW982 |
| | Epithelioid sarcoma | VA-ES-BJ |
| | Ewing sarcoma | A-673 |
| | Embryonal rhabdomyosarcoma | A-204 |
| Esophagus | Squamous Cell Carcinoma | KYSE-150 |
| | Squamous Cell Carcinoma | KYSE-270 |
| | Squamous Cell Carcinoma | KYSE-410 |
| Testis | Testicular embryonal carcinoma | NCC-IT |
| Bone | Osteosarcoma | SJSA1 |
| | Ewing sarcoma | SK-ES-1 |
| | Osteosarcoma | U2OS |
| | Osteosarcoma | 143B |
| Thyroid | Hereditary thyroid gland medullary carcinoma | TT |
| | Anaplastic carcinoma | CAL-62 |
| | Anaplastic carcinoma | 8305 C |

### ^{a)} Therapy-resistant cell lines

### B.1.1 IC50 values in brain cancer cells

The inhibitory activity of the compounds SND462AM, SND462AMH, SND462AMH, and SND631 and comparative compound SND462 in different brain cancer cell lines was tested. The results are summarized in **Table 2.**

As shown by **Table 2,** all tested compounds inhibit the brain cancer cell growth with IC₅₀s lower than or equal to 30nM. The compounds according to the present invention have a significantly better inhibitory activity in brain neuroblastoma cancer cells than known compound SND462 as shown e.g., by the results obtained with Kelly cancer cell line.

**Table 2**

| **Compound** | **Cell line** | **IC₅₀ [NM]** |
|---|---|---|
| SND462 described in WO2022/167698A1* | Kelly | 224 |
| SND462AM | | 3.85 |
| SND462AMH | | 4.92 |
| SND631 | | 0.90 |
| SND462AM | Daoy | 29 |
| SND462AM | IMR-32 | 14.5 |
| SND462AM | LN-229 | 22.3 |
| SND462AM | SUM 159PT | 1.4 |
| SND462AM | U118 MG | 3.8 |
| SND631 | | 14 |
| SND631 | SK-N-AS | 30 |
| SND631 | U87 MG | 11.2 |

| | | |
|---|---|---|
| *for comparative purposes | | |

### B.1.2 IC50 values in bladder cancer cells

The inhibitory activity of the compounds SND462AM, SND462AMH and SND631 in different bladder cancer cell lines was tested. The results are summarized in **Table 3.**

As shown by **Table 3,** all tested compounds inhibit the bladder carcinoma cell growth with IC₅₀s lower than or equal to 40nM. The compounds according to the present invention present improved inhibitory activity compared with known compound SND462 as shown for e.g., by the results obtained with the bladder carcinoma cell line 5637.

**Table 3**

| Compound | Cell line | IC₅₀ [nM] |
|---|---|---|
| SND462 described in WO2022/167698A1* | 5637 | >20000 |
| SND462AM | | 20.4 |
| SND462AMH | | 41 |
| SND631 | | 26 |
| SND462AM | RT112/84 | 7.1 |
| SND462AM | RT-4 | 27.8 |
| SND631 | RT-4 | 39 |
| SND462AM | SW780 | 14.5 |
| SND631 | T24 | 6.8 |

| | | |
|---|---|---|
| *for comparative purposes | | |

### B.1.3 IC50 values in breast cancer cells

The inhibitory activity of the compounds SND462AM, SND462AMH, SND631 and SND632 in different breast cancer cell lines was tested. The results are summarized in **Table 4.**

As shown by **Table 4,** all tested compounds inhibit the breast carcinoma cell growth, including breast aggressive cancer types, such as pleomorphic carcinoma, squamous cell carcinoma, and breast metastatic adenocarcinoma, with IC₅₀s lower than to 40nM. SND462 shows low nanomolar IC₅₀s even in hormone-therapy resistant cell lines MDA-MB-231 and HCC 1806, as well as in trastuzumab-resistant cell line HCC1954.

The compounds according to the present invention present improved inhibitory activity compared with known compound SND462 as shown for e.g., by the results obtained with the breast metastatic carcinoma MDA-MB-468.

**Table 4**

| Compound | Cell line | IC₅₀ [nM] |
|---|---|---|
| SND462 described in WO2022/167698A1* | MDA-MB-468 | >20000 |
| SND462AM | | 1.41 |
| SND462AMH | | 2.32 |
| SND631 | | 0.7 |
| SND632 | | 57.48 |
| SND462AM | MDA-MB-231 | 10.7 |
| SND631 | BT-549 | 31 |
| SND462AM | HCC 1187 | 6.2 |
| SND462AM | HCC 1954 | 33.9 |
| SND462AM | DU4475 | 19.8 |
| SND462AM | HCC 1806 | 11.8 |
| SND462AM | T47D | 41.2 |
| SND462AM | CAL-51 | 7.8 |
| SND631 | BT-549 | 31 |
| SND631 | Hs 578T | 29 |

### B.1.4 IC50 values in blood leukemia cells

The inhibitory activity of the compounds SND462AM, SND462AMH, and SND631, and comparative compound SND462 in different leukemia cell lines was tested. The results are summarized in **Table 5.**

As shown by **Table 5,** all tested compounds inhibit the leukemia cell growth, including in aggressive leukemia types, such as ALL, and therapy-resistant leukemia types (e.g., K-562), with IC₅₀s lower than to 30nM. The compounds according to the present invention present improved inhibitory activity compared with known compound SND462 as shown for e.g., by the results obtained with the chronic myeloid leukemia cell line.

**Table 5**

| Compound | Cell line | IC₅₀ [nM] |
|---|---|---|
| SND462 described in WO2022/167698A1* | K-562 | 152 |
| SND462AM | | 20.8 |
| SND462AM | CCRF-CEM | 14.1 |
| SND631 | | 11 |
| SND462AM | HL-60 | 20.6 |
| SND631 | | 31 |
| SND462AM | RS4;11 | 4.5 |
| SND631 | | 6.5 |
| SND631 | MOLT-4 | 24 |
| SND631 | Jurkat E6.1 | 3.2 |
| SND631 | KG-1 | 25 |
| SND631 | Ku812 | 12 |
| SND462AM | EOL-1 | 4.6 |
| SND462AM | ML-2 | 8.2 |
| SND462AM | JVM-3 | 20.9 |
| SND462AM | NALM-6 | 17.3 |
| SND462AM | OCI-AML3 | 17.7 |
| SND462AM | PEER | 14.2 |

### B.1.5 IC50 values in blood lymphoma cells

The inhibitory activity of the compounds SND462AM, SND462AMH, and SND631, and comparative compound SND462 in different lymphoma cell lines was tested. The results are summarized in **Table 6.**

As shown by **Table 6,** all tested compounds inhibit the leukemia cell growth, including aggressive lymphoma types, with IC₅₀s lower than to 50nM. The compounds according to the present invention present significantly improved inhibitory activity compared with known compound SND462 as shown for e.g., by the results obtained with the B-cell lymphoma cell lines.

**Table 6**

| Compound | Cell line | IC₅₀ [nM] |
|---|---|---|
| SND462 described in WO2022/167698A1* | JEKO1 | 1341.7 |
| SND462AM | | 2.8 |
| SND631 | | 16 |
| SND462 described in WO2022/167698A1* | KARPAS 422 | 974.57 |
| SND462AM | | 7.1 |
| SND462 described in WO2022/167698A1* | Pfeiffer | 3391.19 |
| SND462AM | | 28.5 |
| SND462 described in WO2022/167698A1* | REC1 | 1385.90 |
| SND462AM | | 42.5 |
| SND462 described in WO2022/167698A1* | SU-DHL-4 | 2631.03 |
| SND462AM | | 5.9 |
| SND462AM | RL | 2.5 |
| SND631 | | 5.3 |
| SND462AM | SU-DHL-1 | 5 |
| SND631 | | 22 |
| SND462AM | SU-DHL-2 | 12.7 |
| SND462AM | SU-DHL-8 | 17.2 |
| SND462AM | SU-DHL-10 | 30.8 |
| SND462AM | DB | 18 |
| SND462AM | GRANTA-519 | 3.7 |
| SND462AM | HuT 102 | 14 |
| SND462AM | HuT 78 | 35.8 |
| SND462AM | KARPAS 299 | 10.6 |
| SND462AM | L-82 | 7.3 |
| SND462AM | MOLM-13 | 1.9 |
| SND462AM | MOLM-16 | 13.1 |
| SND462AM | NAWALMA | 5.3 |
| SND462AM | OCI-LY19 | 28.5 |
| SND462AM | OCI-LY7 | 4.7 |
| SND462AM | OCI-LY3 | 14.9 |
| SND462AM | Raji | 6.9 |
| SND462AM | Ramos | 17.1 |
| SND462AM | Toledo | 13 |
| SND462AM | U937 | 19.5 |
| SND462AM | CA46 | 11.8 |
| SND462AM | ARH-77 | 16.7 |
| SND631 | SR | 1.8 |
| SND631 | HT | 14 |

### B.1.6 IC50 values in blood myeloma cells

The inhibitory activity of the SND462AM in different myeloma cell lines was tested. The results are summarized in **Table 7.**

As shown by **Table 7,** SND462AM inhibits the myeloma cell growth, including plasmacytoma cell growth, with IC₅₀s lower than or equal to 30nM. The compound is efficient also against therapy-resistant cancer types, as demonstrated e.g., by the results obtained with dexamethasone-resistant cell line MM.1R.

**Table 7**

| Compound | Cell line | IC₅₀ [nM] |
|---|---|---|
| SND462 described in WO2022/167698A1* | JJN-3 | >20000 |
| SND462AM | | 23.8 |
| SND462AM | KMS-11 | 24.1 |
| SND462AM | L-363 | 21.8 |
| SND462AM | MM.1R | 25 |
| SND462AM | MM.1S | 16 |
| SND462AM | MOLP8 | 17.7 |
| SND462AM | NCI-H929 | 12.2 |
| SND462AM | OPM2 | 7.6 |
| SND462AM | RPMI-8226 | 13.7 |
| SND462AM | U266B1 | 30.3 |
| SND462AM | ALMC-2 | 13.3 |
| SND462AM | ALMC-1 | 14.8 |
| SND462AM | AMO-1 | 30.2 |

### B.1.7 IC50 values in head and neck cancer cells

The inhibitory activity of the SND462AM and SND631 in different head and neck cancer cell lines was tested. The results are summarized in **Table 8.**

As shown by **Table 8,** the tested compounds inhibit head and neck cancer cell growth with IC₅₀s lower than or equal to 50nM. The compounds according to the present invention present significantly improved inhibitory activity compared with known compound SND462 as shown for e.g., by the results obtained with CAL-27 cell line.

**Table 8**

| Compound | Cell line | IC₅₀ [nM] |
|---|---|---|
| SND462 described in WO2022/167698A1* | CAL-27 | >20000 |
| SND462AM | | 34.30 |
| SND631 | | 44 |
| SND462AM | Detroit 562 | 1.6 |
| SND462AM | FaDu | 12.90 |
| SND462AM | SCC-4 | 9.1 |

### B.1.8 IC50 values in kidney carcinoma cells

The inhibitory activity of the SND462AM in kidney cell lines was tested. The results are summarized in **Table 9.**

As shown by **Table 9,** the SND462AM inhibits with kidney cancer cell growth with IC₅₀s lower than or equal to 30nM. The compounds according to the present invention present significantly improved inhibitory activity compared with known compound SND462 as shown for e.g., by the results obtained with the Caki-1 cell line.

**Table 9**

| Compound | Cell line | IC₅₀ [nM] |
|---|---|---|
| SND462 described in WO2022/167698A1* | Caki-1 | >20000 |
| SND462AM | | 2.8 |
| SND462AM | 786-O | 10.8 |
| SND462AM | NCI-H295R | 23.1 |
| SND462AM | ACHN | 10.1 |

### B.1.9 IC50 values in intestinal cancer cells

The inhibitory activity of the compounds SND462AM, SND462AMH, and SND631 in different intestinal cancer cell lines was tested. The results are summarized in **Table 10.**

As shown by **Table 10,** all tested compounds inhibit the intestinal cancer cell growth, with IC₅₀s lower than to 60nM. All compounds are particularly efficient against duodenal carcinoma and colon carcinoma with IC₅₀s values lower than 5 nM. The compounds according to the present invention present significantly improved inhibitory activity compared with known compound SND462 as shown for e.g., by the results obtained with the HuTu 80 and RKO cell lines.

**Table 10**

| Compound | Cell line | IC₅₀ [nM] |
|---|---|---|
| SND462 described in WO2022/167698A1* | HuTu 80 | >20000 |
| SND462AM | | 1.2 |
| SND462AMH | | 2.33 |
| SND631 | | 0.3 |
| SND462AM | COLO 205 | 11.9 |
| SND462AM | DLD-1 | 36.4 |
| SND462AM | HT-29 | 12 |
| SND462AM | HT-55 | 9.8 |
| SND462AM | LoVo | 13.8 |
| SND462AM | LS174T | 20.4 |
| SND462AM | SW1463 | 20.3 |
| SND462AM | SW403 | 37.2 |
| SND462AM | SW948 | 30.8 |
| SND631 | | 55 |
| SND462AM | HCT 116 | 6 |
| SND462AM | LS1034 | 21.4 |
| SND462AM | LS411N | 17.6 |
| SND462AM | NCI-H716 | 35.7 |
| SND631 | SNU-C2B | 45 |
| SND462 described in WO2022/167698A1* | RKO | >20000 |
| SND462AM | | 1 |
| SND462AMH | | 1.40 |
| SND631 | | 1.22 |
| SND462AM | SW626 | 17.9 |
| SND462AM | SNU-81 | 49.6 |
| SND462AM | CCK-81 | 32 |
| SND462AM | SW837 | 14.5 |

### B.1.10 IC50 values in liver carcinoma cells

The inhibitory activity of SND462AM in different liver cancer cell lines was tested. The results are summarized in **Table 11.**

As shown by **Table 11,** SND462AM inhibits the liver cancer cell growth with IC₅₀s lower than to 40nM. The compounds according to the present invention present significantly improved inhibitory activity compared with known compound SND462 as shown for e.g., by the results obtained with Hep G2/C3A cell line.

**Table 11**

| Compound | Cell line | IC₅₀ [nM] |
|---|---|---|
| SND462 described in WO2022/167698A1* | Hep G2/C3A | >20000 |
| SND462AM | | 14.3 |
| SND462AM | Hep3B | 2.7 |
| SND462AM | HepG2 | 9.3 |
| SND462AM | HUH-7 | 2.6 |
| SND462AM | JHH-5 | 30.3 |
| SND462AM | JHH-7 | 33.3 |
| SND462AM | PLC/PRF/5 | 28.9 |

### B.1.11 IC50 values in skin cancer cells

The inhibitory activity of the compounds SND462AM, SND462AMH, and SND631 in different skin melanoma cell lines was tested. The results are summarized in **Table 12.**

As shown by **Table 12,** all tested compounds inhibit the skin melanoma cell growth with IC₅₀s lower than to 40nM. The compounds prove efficient also against targeted-therapy resistant skin melanoma forms (e.g., SK-MEL-2). The compounds are extremely efficient against amelanotic melanoma showing IC₅₀ values lower than 2nM. The compounds according to the present invention present significantly improved inhibitory activity compared with known compound SND462 as shown for e.g., by the results obtained with A-2058 cell line.

**Table 12**

| Compound | Cell line | IC₅₀ [nM] |
|---|---|---|
| SND462 described in WO2022/167698A1* | A-2058 | >20000 |
| SND462AM | | 1.02 |
| SND462AMH | | 1.31 |
| SND631 | G-361 | 38 |
| SND462AM | RPMI-7951 | 3.4 |
| SND462AM | SK-MEL-2 | 8.4 |
| SND462AM | SK-MEL-5 | 21.2 |
| SND462AM | SK-MEL-28 | 37.4 |
| SND462AM | A-375 | 16.3 |

### B.1.12 IC50 values in pancreatic cancer cells

The inhibitory activity of the compounds SND462AM, SND462AMH, SND462AMM, SND631 and SND632 in different pancreatic cancer cell lines was tested. The results are summarized in **Table 13.**

As shown by Table 13, all tested compounds inhibit the pancreatic cancer cell growth with IC₅₀s lower than 100 nM. In particular, SND462AM exhibits IC₅₀s values below 30nM, while SND631 exhibits IC₅₀s lower than 50 nM. The compounds are efficient also against chemotherapy-resistant pancreatic cancer forms (e.g., PANC-1). The compounds according to the present invention present significantly improved inhibitory activity compared with known compound SND462 as shown for e.g., by the results obtained with AsPC-1 and Mia-PaCa-2 cell lines.

**Table 13**

| Compound | Cell line | IC₅₀ [nM] |
|---|---|---|
| SND462 described in WO2022/167698A1* | AsPC-1 | >20000 |
| SND462AM | | 12.2 |
| SND462AMH | | 60 |
| SND631 | | 35 |
| SND462AM | BxPC-3 | 16.6 |
| SND631 | | 49 |
| SND462AM | HPAF-II | 14.8 |
| SND462AM | Hs 766T | 3.6 |
| SND631 | | 36 |
| SND462AM | HuP-T4 | 10 |
| SND462 described in WO2022/167698A1* | Mia-PaCa-2 | >20000 |
| SND462AM | | 2 |
| SND462AMM | | 2.54 |
| SND631 | | 0.3 |
| SND632 | | 86.15 |
| SND462AM | PANC-1 | 14.7 |
| SND462AM | PSN-1 | 20.7 |
| SND462AM | Panc 02.03 | 29.5 |
| SND462AM | Panc 05.04 | 6.1 |
| SND462AM | Panc 03.27 | 8.6 |
| SND462AM | SW1990 | 8.1 |
| SND462AM | Capan-2 | 9.6 |
| SND462AM | HuP-74 | 10 |

### B.1.13 IC50 values in lung carcinoma cells

The inhibitory activity of the compounds SND462AM, SND462AMH, and SND631 in different lung cancer cell lines was tested. The results are summarized in **Table 14.**

As shown by Table 14, all tested compounds inhibit the lung cancer cell growth with IC₅₀s lower than 50 nM. In particular, SND631 exhibits IC₅₀s lower than 30 nM in a variety of lung cancer cells. The compounds prove efficient also against targeted-therapy resistant cancer types (e.g., NCI-H1975). The compounds according to the present invention present significantly improved inhibitory activity compared with known compound SND462 as shown for e.g., by the results obtained with COR-L23, SHP-77 and SW900 cell lines.

**Table 14**

| Compound | Cell line | IC₅₀ [nM] |
|---|---|---|
| SND462 described in WO2022/167698A1* | COR-L23 | >20000 |
| SND462AM | | 25.4 |
| SND462AM | DMS-114 | 27.7 |
| SND462AM | DMS-53 | 8.9 |
| SND462AM | HCC 95 | 31.1 |
| SND462AM | EBC-1 | 45.20 |
| SND462AM | HCC 4006 | 15.3 |
| SND462AM | HCC 515 | 20.5 |
| SND462AM | HCC 827 | 12.8 |
| SND462AM | HCC 2935 | 16.9 |
| SND462AM | LC-2/AD | 10.3 |
| SND462AM | LCLC-97TM1 | 13 |
| SND462AM | NCI-H1299 | 9.1 |
| SND462AM | NCI-H1395 | 4.7 |
| SND462AM | NCI-H1563 | 10.3 |
| SND462AM | NCI-H1568 | 10.7 |
| SND462AM | NCI-H1573 | 4.8 |
| SND462AM | NCI-H1581 | 11.5 |
| SND462AM | NCI-H1650 | 7.7 |
| SND462AM | NCI-H1703 | 23.6 |
| SND462AM | NCI-H1688 | 32 |
| SND462AM | NCI-H1666 | 32.4 |
| SND462AM | NCI-H1781 | 11.2 |
| SND462AM | NCI-H187 | 2.9 |
| SND462AM | NCI-H1975 | 12.2 |
| SND462AM | NCI-H1792 | 34.3 |
| SND462AM | NCI-H2052 | 8 |
| SND462AM | NCI-H2110 | 9.5 |
| SND462AM | NCI-H209 | 30.5 |
| SND462AM | NCI-H23 | 11.2 |
| SND462AM | NCI-H2228 | 26.8 |
| SND462AM | NCI-H292 | 32.5 |
| SND462AM | NCI-H441 | 12.8 |
| SND462AM | NCI-H446 | 14.9 |
| SND462AM | NCI-H520 | 7.3 |
| SND462AM | NCI-H460 | 13.6 |
| SND631 | NCI-H661 | 24 |
| SND462AM | NCI-H526 | 24.7 |
| SND462AM | NCI-H727 | 24.3 |
| SND462AM | NCI-H838 | 22.1 |
| SND462 described in WO2022/167698A1* | SHP-77 | >20000 |
| SND631 | | 9.1 |
| SND462AM | | 37.3 |
| SND462AM | SNU-878 | 35.9 |
| SND462 described in WO2022/167698A1* | SW900 | >20000 |
| SND631 | | 20 |
| SND462AM | | 20.4 |
| SND462AM | CALU-6 | 1.9 |
| SND462AM | CALU-1 | 6.8 |
| SND462AM | A-549 | 9.6 |
| SND462AM | A-427 | 1 |
| SND631 | | 14 |
| SND462AM | PC-9 | 21.2 |
| SND462AM | CALU-3 | 24.9 |

### B.1.14 IC50 values in gynecologic cancer cells

The inhibitory activity of the compounds SND462AM, and SND631 in different gynecologic cancer cell lines was tested. The results are summarized in **Table 15.**

As shown by Table 15, all tested compounds inhibit the gynecologic cancer cell growth with IC₅₀s lower than 50 nM. The compounds are efficient also against therapy-resistant cancer types (e.g., multi-drug resistant SK-OV-3 cancer cell line, cis-platin resistant cancer cell lines OVCAR-3 and A2780cis). The compounds exhibit IC₅₀s lower than 1nM against ovarian teratocarcinoma, lower than 20nM against uterine choriocarcinoma, and endometrial adenocarcinoma and lower than 20nM against a variety of ovarian adenocarcinomas. The compounds according to the present invention present significantly improved inhibitory activity compared with known compound SND462 as shown for e.g., by the results obtained with ES-2 cell line.

**Table 15**

| Compound | Cell line | IC₅₀ [nM] |
|---|---|---|
| SND462AM | AN3-CA | 15.30 |
| SND462AM | Ishikawa | 1.8 |
| SND631 | KLE | 13 |
| SND462AM | COV504 | 22.6 |
| SND462 described in WO2022/167698A1* | ES-2 | >20000 |
| SND462AM | | 0.3 |
| SND631 | | 3.4 |
| SND462AM | IGR-OV1 | 31.2 |
| SND462AM | OVCAR-3 | 15.7 |
| SND462AM | OVCAR-4 | 14.9 |
| SND462AM | OVCAR-5 | 17.1 |
| SND462AM | OVCAR-8 | 19.8 |
| SND631 | RL95-2 | 18 |
| SND462AM | RMG-1 | 19.3 |
| SND462AM | SK-OV-3 | 7.7 |
| SND462AM | TOV-21G | 40.4 |
| SND462AM | A2780 | 15.4 |
| SND462AM | A2780cis | 19.1 |
| SND462AM | RMG-1 | 19.3 |
| SND462AM | HeLa | 11.5 |
| SND462AM | JAR | 8 |
| SND631 | | 24 |
| SND462AM | JEG-3 | 15.2 |
| SND462AM | PA-1 | 0.86 |
| SND631 | | 0.90 |

### B.1.15 IC50 values in prostate cancer cells

The inhibitory activity of the compounds SND462AM and SND631 in different prostate cancer cell lines was tested. The results are summarized in **Table 16.**

As shown by Table 16, all tested compounds inhibit the prostate cancer cell growth with IC₅₀s lower than 70 nM. The compounds are efficient also against therapy-resistant prostate cancer types, including hormone-therapy resistant prostate cancer types (e.g., DU145, PC-3). Further, SND462AM exhibits IC₅₀s lower than 40nM against all tested prostate cancer cell lines. The compounds according to the present invention present significantly improved inhibitory activity compared with known compound SND462 as shown for e.g., by the results obtained with DU145 cell line.

**Table 16**

| Compound | Cell line | IC₅₀ [nM] |
|---|---|---|
| SND462 described in WO2022/167698A1* | DU145 | >20000 |
| SND462AM | | 6.6 |
| SND631 | | 69 |
| SND462AM | NCI-H660 | 35 |
| SND462AM | PC-3 | 10.6 |
| SND462AM | 22Rv1 | 31.5 |

### B.1.16 IC50 values in stomach cancer cells

The inhibitory activity of the compounds SND462AM and SND631 in different stomach cancer cell lines was tested. The results are summarized in **Table 17.**

As shown by Table 17, all tested compounds inhibit the stomach cancer cell growth with IC₅₀s lower than 30 nM. The compounds according to the present invention present significantly improved inhibitory activity compared with known compound SND462 as shown for e.g., by the results obtained with HGC-27 cell line.

**Table 17**

| Compound | Cell line | IC₅₀ [nM] |
|---|---|---|
| SND462 described in WO2022/167698A1* | HGC-27 | >20000 |
| SND462AM | | 17 |
| SND462AM | HuCCT1 | 12.4 |
| SND631 | Hs 746.T | 6.6 |
| SND462AM | MKN1 | 12 |
| SND462AM | SNU-620 | 16.3 |
| SND462AM | SNU-16 | 27.4 |
| SND462AM | SNU-601 | 34.2 |
| SND462AM | YCC-2 | 9.4 |
| SND462AM | YCC-10 | 15.8 |

### B.1.17 IC50 values in soft tissue (muscle) cancer cells

The inhibitory activity of the compounds SND462AM and SND631 in different muscle cancer cell lines was tested. The results are summarized in **Table 18.**

As shown by Table 18, all tested compounds inhibit the soft tissue cancer cell growth with IC₅₀s lower than 50 nM. The compounds according to the present invention present significantly improved inhibitory activity compared with known compound SND462 as shown for e.g., by the results obtained with HT-1080 cell line.

**Table 18**

| Compound | Cell line | IC₅₀ [nM] |
|---|---|---|
| SND462 described in WO2022/167698A1* | HT-1080 | >20000 |
| SND462AM | | 2.2 |
| SND631 | | 44 |
| SND631 | SW982 | 28 |
| SND631 | RD | 23 |
| SND631 | A-204 | 25 |
| SND631 | Rh30 | 20 |
| SND631 | VA-ES-BJ | 36 |
| SND462AM | SJCRH30 | 5.6 |
| SND462AM | A-673 | 9.80 |
| SND462AM | SW872 | 22 |

### B.1.18 IC50 values in esophageal cancer cells

The inhibitory activity of SND462AM in different esophageal cancer cell lines was tested. The results are summarized in **Table 19.**

As shown by Table 19, SND462AM inhibits the esophageal cancer cell growth with IC₅₀s lower than 20 nM. The compounds according to the present invention present significantly improved inhibitory activity compared with known compound SND462 as shown for e.g., by the results obtained with KYSE-150 cell line.

**Table 19**

| Compound | Cell line | IC₅₀ [nM] |
|---|---|---|
| SND462 described in WO2022/167698A1* | KYSE-150 | >20000 |
| SND462AM | | 15.9 |
| SND462AM | KYSE-270 | 5.7 |
| SND462AM | KYSE-410 | 13.9 |

### B.1.19 IC50 values in testicular cancer cells

The inhibitory activity of SND462AM in testicular cancer cells was tested. The results are summarized in **Table 20.**

As shown by Table 20, SND462AM inhibits the testicular cancer cell growth with an IC₅₀ of 16 nM. The compounds according to the present invention present significantly improved inhibitory activity compared with known compound SND462 as shown for e.g., by the results obtained with NCC-IT cell line.

**Table 20**

| Compound | Cell line | IC₅₀ [nM] |
|---|---|---|
| SND462 described in WO2022/167698A1* | NCC-IT | >20000 |
| SND462AM | | 16 |

### B.1.20 IC50 values in bone cancer cells

The inhibitory activity of SND462AM in different bone cancer cell lines was tested. The results are summarized in **Table 21.**

As shown by Table 21, SND462AM inhibits the bone cancer cell growth with IC₅₀s lower than 20 nM. The compounds according to the present invention present significantly improved inhibitory activity compared with known compound SND462 as shown for e.g., by the results obtained with SJSA1 cell line.

**Table 21**

| Compound | Cell line | IC₅₀ [nM] |
|---|---|---|
| SND462 described in WO2022/167698A1* | SJSA1 | >20000 |
| SND462AM | | 12.4 |
| SND462AM | 143B | 3 |
| SND631 | U2OS | 10 |
| SND462AM | SK-ES-1 | 13.3 |

### B.1.21 IC50 values in thyroid cancer cells

The inhibitory activity of SND462AM in different thyroid cancer cell lines was tested. The results are summarized in **Table 22.**

As shown by Table 22, SND462AM inhibits the thyroid cancer cell growth with IC₅₀s lower than 10 nM. The compounds according to the present invention present significantly improved inhibitory activity compared with known compound SND462 as shown for e.g., by the results obtained with CAL-62 cell line.

**Table 22**

| Compound | Cell line | IC₅₀ [nM] |
|---|---|---|
| SND462 described in WO2022/167698A1* | CAL-62 | >20000 |
| SND462AM | | 1.6 |
| SND462AM | 8305 C | 2.5 |
| SND462AM | TT | 9.5 |

### B.2 Evaluation of anti-proliferative effect of the compounds SND631, SND635, and and SND644 in five cancer cell lines

The antitumor activity of the compounds SND631, SND635, and SND644 in 5 cell lines (Mia-Pa-ca 2, SU-DHL 6, PA-1, MDA-MB-468, A-2058) was assessed by using CellTiter-Glo Luminescent Cell Viability Assay kit (Promega-G7573) according to the manufacturer's protocol and EnVision Multilabel Reader (PerkinElmer). Paclitaxel was used as a positive control.

### Materials

**Table 23: Cell lines and propagation**

| **Cell Line** | **Cancer Type** | **Vendor** | **Cat. No.** | **Culture Property** | **Culture Medium** |
|---|---|---|---|---|---|
| Mia-paca-2 | Pancreatic cancer | ATCC | CRL-1420 | Adherent, single cells and loosely attached clusters | DMEM/F12 + 10% FBS |
| PA-1 | Ovarian mixed germ cell tumor | ATCC | CRL-1572 | Adherent | EMEM+10% hiFBS |
| SU-DHL-6 | Lymphoma | ATCC | CRL-2959 | Suspension | RPMI 1640 + 10% FBS |
| MDA-MB-468 | Breast cancer | ATCC | HTB-132 | Adherent | L-15 + 10% FBS |
| A2058 | Amelanotic melanoma | ATCC | CRL-11147 | Adherent | DMEM + 10% FBS |

**Table 24: Media and reagents**

| **Media and Reagents** | **Vendor** | **Catalog No.** |
|---|---|---|
| Antibiotic-Antimycotic | Gibco | 15240-062 |
| FBS | Excell Bio | FSP500 |
| 0.25% Trypsin | Gibco | 25200-072 |
| Dulbecco's PBS | Corning | 21-031-CVC |
| DMSO | SIGMA | D2650 |
| RPMI 1640 | Gibco | 22400-089 |
| EMEM | ATCC | 30-2003 |
| DMEM | Gibco | 11965-092 |
| DMEM/F12 | Gibco | 11330032 |
| L15 | SIGMA | L-1518 |

Assay plate: Greiner CELLSTAR^{®} 96 well plates with black wells flat bottom (with lid and micro-clear bottom), # 655090.

Compound plate: Nunc-442587, Pinchbar Design Polypropylene, V-shape well bottoms, Sterile.B

### Anti-proliferation assay

### Cell culture

All cells were maintained in culture conditions at 37°C in an atmosphere with 5%CO₂ in air. The tumor cells were routinely subcultured. The cells growing in an exponential growth phase were harvested and counted for plating.

### Cell Plating

(1). Count cells by haemocytometer with Trypan blue staining.
(2). Adjust the cell concentration to proper cell density.

| **NO.** | **Cell name** | **Density (per 96-well)** |
|---|---|---|
| **1** | PA-1 | 3000 |
| **2** | Mia-PaCa-2 | 2000 |
| **3** | A2058 | 2000 |
| **4** | MDA-MB-468 | 5000 |

(3). Plate 90 µL of cell suspension into the assay plates. Add 90 µL of assay medium into the Blank wells.
(4). Incubate the plates at 37°C, 5% CO₂, 95% air and 100% relative humidity overnight.

### Compound Plate Preparation and Cell Viability Measurement

A 9-point dilution series for each tested compound was obtained by diluting the tested compound in DMSO with 10-fold steps from a 1000 nM stock and a 31.6-fold dilution in Dulbecco PBS (pH 7.4).

The dilution series for each tested compound was added in triplicate to the cells in the 96-well assay plates to determine the effect of the tested compound on cell viability. For vehicle and blank group DMSO-medium was added into the wells. The final DMSO concentration was concentration was 0.1% (v/v). The cell viability was measured in each well after 72 hours of incubation with compound following manufacturer's protocol.

### Data Analysis

Inhibition rate (IR) of the tested compounds was determined by the following formula: IR (%) = (1- (RLU compound - RLU blank) / (RLU control - RLU blank))*100%. The inhibitions of different dose of compound were calculated, and then were used to plot inhibition curves and evaluate related parameters, such as Min (%), Max (%) and IC50. The data were interpreted by GraphPad Prism.

The IC50 values are depicted in **Table 25.**

As shown in **Table 25** the compounds SND631, SND635 and SND644 exhibit significantly lower IC₅₀ values and better inhibitory activity than Paclitaxel in Mia-Paca-2 pancreatic cancer cell line. Further SND631 and SND635 exhibit significantly lower IC₅₀ values and improved inhibitory activity than Paclitaxel in PA-1, A-2058 and MDA-MB-468 cell lines.

**Table 25: IC50 value (nM) in different cell lines**

| Cell line | Cancer type | Compound | Absolute IC50 (nM) |
|---|---|---|---|
| Mia-PaCa-2 | Pancreatic | SN D644 | 0.420 |
| | | SND631 | 0.484 |
| | | SND635 | 0.161 |
| | | Paclitaxel | 9.961 |
| PA-1 | Ovarian | SND644 | 69.354 |
| | | SND631 | 0.062 |
| | | SND635 | 0.665 |
| | | Paclitaxel | 1.955 |
| MDA-MB-468 | Breast | SN D644 | 25.796 |
| | | SND631 | 0.427 |
| | | SND635 | 0.660 |
| | | Paclitaxel | 6.061 |
| A-2058 | Melanoma | SN D644 | 23.226 |
| | | SND631 | 1.046 |
| | | SND635 | 0.543 |
| | | Paclitaxel | 2.317 |

### B.3 Comparison of anti-proliferative effects of SND462AM and Ibutrinib in different cancer cell lines

The IC50 values of SND462AM and Ibutrinib, a well-known and intensively used anticancer drug, were measured using the methodology described at section B.1 in different cancer cells. The results are depicted in Table 26.

**Table 26: IC50 value (µM) in different cell lines**

| **Cancer cell line** | **IC50 SND462AM (µM)** | **IC50 Ibutrinib (µM)** |
|---|---|---|
| 5637 | 0.0204 | 48.911583 |
| a2780 | 0.0154 | 44.510878 |
| a549 | 0.0096 | 49.059309 |
| achn | 0.0101 | 11.940132 |
| aqs | 0.0689 | 126.891669 |
| AMO-1 | 0.0302 | 27.022793 |
| ARH-77 | 0.0167 | 52.704627 |
| aspc-1 | 0.0122 | 2517.18358 |
| bt-474 | 0.0967 | 3.417412 |
| bxpc-3 | 0.0166 | 101.52867 |
| ca46 | 0.0118 | 97.771793 |
| caki-1 | 0.0028 | 4.797025 |
| cal-27 | 0.0343 | 4.781612 |
| cal-51 | 0.0078 | 250.059974 |
| cal-62 | 0.0016 | 207.350698 |
| calu-3 | 0.0249 | 0.928269 |
| calu-6 | 0.0019 | 587.414135 |
| capan-1 | 0.1093 | 96.936805 |
| capan-2 | 0.0096 | 890.742929 |
| cck-81 | 0.032 | 5.598461 |
| ccrf-cem | 0.0141 | 68.23397 |
| cfpac-1 | 0.0744 | 151.491966 |
| cor-l23 | 0.0254 | 666.612836 |
| daudi | 0.0123 | 110.33858 |
| ebc-1 | 0.0452 | 84.377933 |
| es-2 | 0.0003 | 258.163383 |
| fadu | 0.0129 | 6.733984 |
| qranta-519 | 0.0037 | 312.487353 |
| hct-15 | 0.389 | 77.914108 |
| hela | 0.0115 | 155.118349 |
| hgc-27 | 0.017 | 18.122879 |
| hl-60 | 0.0206 | 25.799799 |
| hpaf-ii | 0.0148 | 588.080601 |
| ht-1080 | 0.0022 | 479.460667 |
| ht-29 | 0.012 | 60.890544 |
| huh-1 | 0.4944 | 227.590956 |
| huh-7 | 0.0026 | 299.942848 |
| hup-t4 | 0.01 | 334.467325 |
| jar | 0.008 | 19.251416 |
| jeg-3 | 0.0152 | 45.386051 |
| jeko-1 | 0.0028 | 1.541976 |
| jhh-7 | 0.0333 | 99.679025 |
| jjn-3 | 0.0238 | 96.857435 |
| jvm-3 | 0.0209 | 14.995248 |
| k-562 | 0.0208 | 30.221461 |
| karpas-299 | 0.0106 | 44.542122 |
| karpas-422 | 0.0071 | 4.163654 |
| kasumi-1 | 0.0436 | 6.131568 |
| kms-11 | 0.0241 | 20.817659 |
| kyse-270 | 0.0057 | 14.009027 |
| kyse-410 | 0.0139 | 35.83062 |
| l-363 | 0.0218 | 82.511402 |
| lclc-97tm1 | 0.013 | 136.613975 |
| In-229 | 0.0223 | 87.113458 |
| lovo | 0.0138 | 15.61561 |
| lp-1 | 0.0605 | 206.294787 |
| mcf7 | 0.9316 | 499.715967 |
| mda-mb-231 | 0.0107 | 68.67629 |
| mkn1 | 0.012 | 94.608186 |
| mkn45 | 0.0569 | 546.275355 |
| ml-2 | 0.0082 | 99.282231 |
| molm-13 | 0.0019 | 1.190598 |
| molm-16 | 0.0131 | 25.291874 |
| molt-4 | 0.0638 | 117.595864 |
| nalm-6 | 0.0173 | 21.186444 |
| namalwa | 0.0053 | 6.967298 |
| nci-h1048 | 0.0434 | 191.790007 |
| nci-h1155 | 0.068 | 78.182531 |
| nci-h1299 | 0.0091 | 454.78455 |
| nci-h1435 | 0.0522 | 138.738979 |
| nci-h146 | 0.5539 | 58.28223 |
| nci-h1563 | 0.0103 | 227.610302 |
| nci-h1568 | 0.0107 | 4.496883 |
| nci-h1581 | 0.0115 | 13.761807 |
| nci-h1650 | 0.0077 | 181.006288 |
| nci-h1651 | 0.0843 | 193.790966 |
| nci-h1666 | 0.0324 | 24.559777 |
| nci-h1781 | 0.0112 | 1.858054 |
| nci-h1792 | 0.0343 | 177.21099 |
| nci-h1836 | 0.1238 | 467.179201 |
| nci-h1975 | 0.0122 | 2.322383 |
| nci-h1993 | 0.031 | 377.272599 |
| nci-h2023 | 0.0359 | 41.30404 |
| nci-h2030 | 0.0108 | 8.455221 |
| nci-h2052 | 0.008 | 332.232794 |
| nci-h209 | 0.0305 | 152.328047 |
| nci-h2110 | 0.0095 | 13.623758 |
| nci-h2122 | 0.1082 | 18.890857 |
| nci-h2170 | 0.09 | 0.600657 |
| nci-h2228 | 0.0268 | 526.121115 |
| nci-h226 | 0.198 | 1490.76278 |
| nci-h23 | 0.0112 | 119.072894 |
| nci-h292 | 0.0325 | 0.474224 |
| nci-h345 | 9 | 539.751228 |
| nci-h358 | 0.0743 | 24.25925 |
| nci-h441 | 0.0128 | 2726.49489 |
| nci-h446 | 0.0149 | 77.338144 |
| nci-h460 | 0.0136 | 23.904991 |
| nci-h520 | 0.0073 | 109.87001 |
| nci-h526 | 0.0247 | 138.068807 |
| nci-h660 | 0.035 | 210.333825 |
| nci-h716 | 0.0357 | 3.439785 |
| nci-h727 | 0.0243 | 578.042756 |
| nci-h82 | 0.0165 | 35.128263 |
| nci-h838 | 0.0221 | 101.698221 |
| nci-h929 | 0.0122 | 121.345121 |
| nci-n87 | 0.0968 | 0.030705 |
| nuqc-4 | 0.0475 | 12.729163 |
| oci-aml3 | 0.0177 | 88.606602 |
| oci-ly7 | 0.0047 | 11.280015 |
| oe19 | 0.0657 | 0.636427 |
| ov-90 | 4.2643 | 1056.01795 |
| ovcar-3 | 0.0157 | 129.113401 |
| ovcar-4 | 0.0149 | 129.583376 |
| ovcar-5 | 0.0171 | 1086.76712 |
| ovcar-8 | 0.0198 | 83.802942 |
| pc-3 | 0.0106 | 707.372365 |
| raji | 0.0069 | 115.63451 |
| rko | 0.1066 | 260.291714 |
| rl | 0.0025 | 3.053085 |
| rpmi-7951 | 0.0034 | 186.163181 |
| rpmi-8226 | 0.0137 | 20.741943 |
| rt4 | 0.0278 | 87.348725 |
| scc-4 | 0.0091 | 98.959944 |
| sf268 | 0.0609 | 380.125356 |
| shp-77 | 0.0373 | 170.073211 |
| siha | 0.0419 | 349.084113 |
| sk-es-1 | 0.0133 | 79.849091 |
| sk-mel-2 | 0.0084 | 224.7871 |
| sk-mel-28 | 0.0374 | 226.891176 |
| sk-mel-5 | 0.0212 | 141.541166 |
| sk-mes-1 | 0.0597 | 12.013228 |
| sk-ov-3 | 0.0077 | 135.359791 |
| snu-1 | 0.2156 | 194.577157 |
| snu-5 | 9 | 101.67149 |
| snu-81 | 0.0496 | 142.270333 |
| su-dhl-1 | 0.005 | 117.637641 |
| su-dhl-10 | 0.0308 | 5.54488 |
| su-dhl-4 | 0.0059 | 3.216139 |
| su-dhl-6 | 0.0052 | 1.729408 |
| su-dhl-8 | 0.0172 | 68.639882 |
| t47d | 0.0412 | 43.54636 |
| t84 | 0.1186 | 206.234993 |
| thp-1 | 0.7708 | 53.18031 |
| tov-21g | 0.0404 | 90.001421 |

### B.4 Comparison of anti-proliferative effects of SND462AM and cis-platin in different cancer cell lines

The IC50 values of SND462AM and cis-platin were measured using the methodology described at section B.1 in different cancer cells. The results are depicted in Table 27.

**Table 27: IC50 value (µM) in different cell lines**

| **Cancer cell line** | **IC50 SND462AM (µM)** | **IC50 Cis-platin (µM)** |
|---|---|---|
| 5637 | 0.0204 | 1.9301 |
| 143B | 0.003 | 0.5874 |
| 22Rv-1 | 0.0315 | 4.3165 |
| 786-O | 0.0108 | 1.7761 |
| 8305 C | 0.0025 | 5.0689 |
| A 498 | 0.0156 | 4.781 |
| A-375 | 0.0163 | 1.5906 |
| A-427 | 0.001 | 7.0682 |
| A-431 | 0.0662 | 3.9918 |
| A-673 | 0.0098 | 0.3661 |
| A2780 | 0.0154 | 0.7887 |
| A2780 cis | 0.0191 | 7.581 |
| A549 | 0.0096 | 4.9186 |
| ACHN | 0.0101 | 2.0047 |
| AGS | 0.0689 | 9.0813 |
| ALMC-1 | 0.0148 | 1.0477 |
| ALMC-2 | 0.0133 | 1.5602 |
| AMO-1 | 0.0302 | 0.8124 |
| AN3-CA | 0.0153 | 6.2966 |
| ARH-77 | 0.0167 | 0.6647 |
| AsPC-1 | 0.0122 | 3.9281 |
| BT-474 | 0.0967 | 73.5915 |
| BxPC-3 | 0.0166 | 2.1593 |
| CA46 | 0.0118 | 2.6598 |
| Caki-1 | 0.0028 | 2.7925 |
| CAL-148 | 0.3313 | 7.356 |
| CAL-27 | 0.0343 | 2.4848 |
| CAL-51 | 0.0078 | 1.3239 |
| CAL-62 | 0.0016 | 3.1109 |
| Calu-1 | 0.0068 | 10.8555 |
| Calu-3 | 0.0249 | 4.6721 |
| Calu-6 | 0.0019 | 1.3186 |
| Capan-1 | 0.1093 | 1.2555 |
| Capan-2 | 0.0096 | 58.2913 |
| CCK-81 | 0.032 | 11.063 |
| CCRF-CEM | 0.0141 | 0.8712 |
| CFPAC-1 | 0.0744 | 3.4203 |
| COLO 205 | 0.0119 | 5.8103 |
| COLO 320DM | 0.0911 | 5.2059 |
| COR-L23 | 0.0254 | 3.437 |
| COV504 | 0.0226 | 10.1611 |
| Daudi | 0.0123 | 0.6998 |
| Detroit 562 | 0.0016 | 3.9999 |
| DLD-1 | 0.0364 | 5.0175 |
| DMS 114 | 0.0277 | 2.1316 |
| DMS 53 | 0.0089 | 2.754 |
| DU145 | 0.0066 | 1.0244 |
| DU4475 | 0.0198 | 0.7257 |
| EBC-1 | 0.0452 | 12.6564 |
| EOL-1 | 0.0046 | 0.6157 |
| ES-2 | 0.0003 | 1.8757 |
| FaDu | 0.0129 | 2.8322 |
| GP2d | 0.171 | 25.4597 |
| GRANTA-519 | 0.0037 | 1.1267 |
| H69AR | 0.0467 | 7.3207 |
| HCC 1187 | 0.0062 | 1.7805 |
| HCC 1569 | 0.1473 | 5.2157 |
| HCC 1806 | 0.0118 | 1.9045 |
| HCC 1954 | 0.0339 | 8.6595 |
| HCC 2935 | 0.0169 | 52.6 |
| HCC 4006 | 0.0153 | 21.4474 |
| HCC 515 | 0.0205 | 4.604 |
| HCC 827 | 0.0128 | 9.8405 |
| HCC 95 | 0.0311 | 9.9024 |
| HCT 116 | 0.006 | 2.8348 |
| HCT-15 | 0.389 | 6.4453 |
| HeLa | 0.0115 | 0.3294 |
| Hep G2 | 0.0093 | 7.2539 |
| Hep G2/C3A | 0.0143 | 3.7241 |
| Hep3B | 0.0027 | 7.0026 |
| HGC-27 | 0.017 | 3.3662 |
| HL-60 | 0.0206 | 1.3208 |
| HPAF-II | 0.0148 | 11.3263 |
| Hs 766T | 0.0036 | 21.1826 |
| HT-1080 | 0.0022 | 1.1986 |
| HT-1376 | 1.6094 | 8.6368 |
| HT-29 | 0.012 | 13.4237 |
| HT-55 | 0.0098 | 10.8469 |
| HuCCT1 | 0.0124 | 9.7428 |
| HUH-1 | 0.4944 | 5.9843 |
| HUH-7 | 0.0026 | 2.9335 |
| HuP-T4 | 0.01 | 1.2503 |
| HuT 102 | 0.014 | 1.3058 |
| HuT 78 | 0.0358 | 0.4456 |
| IGR-OV1 | 0.0313 | 1.0475 |
| IMR-32 | 0.0145 | 0.1103 |
| Ishikawa | 0.0018 | 2.7834 |
| JAR | 0.008 | 0.7308 |
| JEG-3 | 0.0152 | 2.411 |
| JeKo-1 | 0.0028 | 1.6628 |
| JHH-5 | 0.0303 | 2.1694 |
| JHH-7 | 0.0333 | 7.5519 |
| JJN-3 | 0.0238 | 2.3505 |
| Jurkat E6-1 | 0.0481 | 0.8402 |
| JVM-3 | 0.0209 | 0.6151 |
| K-562 | 0.0208 | 5.7416 |
| KARPAS 299 | 0.0106 | 0.8212 |
| KARPAS 422 | 0.0071 | 4.4707 |
| Kasumi-1 | 0.0436 | 3.9244 |
| KMS-11 | 0.0241 | 3.1101 |
| KPL-4 | 0.0996 | 3.6636 |
| KYSE-150 | 0.0159 | 3.6693 |
| KYSE-270 | 0.0057 | 3.036 |
| KYSE-410 | 0.0139 | 9.3964 |
| L-363 | 0.0218 | 1.7534 |
| L-82 | 0.0073 | 0.8243 |
| LC-2/AD | 0.0103 | 6.5012 |
| LCLC-97TM1 | 0.013 | 5.6256 |
| LN-229 | 0.0223 | 8.954 |
| LNCaP FGC | 0.0677 | 5.3934 |
| LoVo | 0.0138 | 6.3695 |
| LP-1 | 0.0605 | 8.7813 |
| LS1034 | 0.0214 | 11.0924 |
| LS174T | 0.0204 | 2.071 |
| LS180 | 0.0512 | 7.2571 |
| LS411N | 0.0176 | 8.3997 |
| LS 513 | 0.046 | 9.8536 |
| MCF7 | 0.9316 | 17.8237 |
| MDA-MB-231 | 0.0107 | 71.2345 |
| MeWo | 0.1881 | 4.8855 |
| MKN1 | 0.012 | 3.3349 |
| MKN45 | 0.0569 | 3.7274 |
| ML-2 | 0.0082 | 0.8724 |
| MM.1R | 0.025 | 1.047 |
| MM.1S | 0.016 | 1.5997 |
| MOLM-13 | 0.0019 | 0.3931 |
| MOLM-16 | 0.0131 | 4.0619 |
| MOLP8 | 0.0177 | 0.9896 |
| Molt-4 | 0.0638 | 0.5213 |
| MP46 | 0.1181 | 11.0125 |
| NALM-6 | 0.0173 | 1.2016 |
| NAMALWA | 0.0053 | 0.2146 |
| NCI-H1048 | 0.0434 | 2.3873 |
| NCI-H1155 | 0.068 | 2.0069 |
| NCI-H1299 | 0.0091 | 5.1291 |
| NCI-H1373 | 0.0115 | 7.1985 |
| NCI-H1395 | 0.0047 | 6.6678 |
| NCI-H1435 | 0.0522 | 25.6759 |
| NCI-H146 | 0.5539 | 5.0255 |
| NCI-H1563 | 0.0103 | 31.0563 |
| NCI-H1568 | 0.0107 | 1.1117 |
| NCI-H1573 | 0.0048 | 10.5089 |
| NCI-H1581 | 0.0115 | 12.4389 |
| NCI-H1650 | 0.0077 | 5.9951 |
| NCI-H1651 | 0.0843 | 1.4932 |
| NCI-H1666 | 0.0324 | 10.1143 |
| NCI-H1688 | 0.032 | 11.5189 |
| NCI-H1703 | 0.0236 | 12.13 |
| NCI-H1781 | 0.0112 | 2.468 |
| NCI-H1792 | 0.0343 | 2.2589 |
| NCI-H1836 | 0.1238 | 14.4688 |
| NCI-H187 | 0.0029 | 0.5578 |
| NCI-H1975 | 0.0122 | 5.5908 |
| NCI-H1993 | 0.031 | 23.9179 |
| NCI-H2009 | 0.3274 | 26.3345 |
| NCI-H2023 | 0.0359 | 1.5956 |
| NCI-H2030 | 0.0108 | 5.0502 |
| NCI-H2052 | 0.008 | 5.953 |
| NCI-H209 | 0.0305 | 0.2368 |
| NCI-H2110 | 0.0095 | 2.6431 |
| NCI-H2122 | 0.1082 | 14.035 |
| NCI-H2170 | 0.09 | 1.4329 |
| NCI-H2228 | 0.0268 | 8.326 |
| NCI-H226 | 0.198 | 6.9756 |
| NCI-H23 | 0.0112 | 2.1363 |
| NCI-H292 | 0.0325 | 3.0342 |
| NCI-H295R | 0.0231 | 13.697 |
| NCI-H322 | 0.2302 | 9.8043 |
| NCI-H345 | >9 | 48.2177 |
| NCI-H358 | 0.0743 | 10.2593 |
| NCI-H441 | 0.0128 | 16.5546 |
| NCI-H446 | 0.0149 | 2.5016 |
| NCI-H460 | 0.0136 | 0.8098 |
| NCI-H520 | 0.0073 | 7.2788 |
| NCI-H526 | 0.0247 | 0.257 |
| NCI-H660 | 0.035 | 2.5365 |
| NCI-H69 | 0.3882 | 3.9524 |
| NCI-H716 | 0.0357 | 10.9113 |
| NCI-H727 | 0.0243 | 19.1161 |
| NCI-H82 | 0.0165 | 1.5133 |
| NCI-H820 | 0.011 | 3.0914 |
| NCI-H838 | 0.0221 | 2.4811 |
| NCI-H889 | 3.5659 | 6.1729 |
| NCI-H929 | 0.0122 | 5.2396 |
| NCI-N87 | 0.0968 | 3.6593 |
| NUGC-4 | 0.0475 | 4.3186 |
| OCI-AML3 | 0.0177 | 0.9246 |
| OCI-LY19 | 0.0285 | 0.1086 |
| OCI-LY3 | 0.0149 | 0.3664 |
| OCI-LY7 | 0.0047 | 2.3461 |
| OE19 | 0.0657 | 21.2411 |
| OPM2 | 0.0076 | 2.9625 |
| OV-90 | 4.2643 | 6.0252 |
| OVCAR-3 | 0.0157 | 3.5168 |
| OVCAR-4 | 0.0149 | 1.9577 |
| OVCAR-5 | 0.0171 | 3.2671 |
| OVCAR-8 | 0.0198 | 5.5317 |
| Panc 02.03 | 0.0295 | 14.9296 |
| Panc 03.27 | 0.0086 | 9.1278 |
| Panc 04.03 | 0.1196 | 15.1831 |
| Panc 05.04 | 0.0061 | 5.9516 |
| Panc 08.13 | 5.9645 | 5.2088 |
| PANC-1 | 0.0147 | 9.9271 |
| PC-3 | 0.0106 | 5.3608 |
| PC-9 | 0.0212 | 4.0157 |
| PEER | 0.0142 | 3.7327 |
| Pfeiffer | 0.0285 | 3.2589 |
| PLC/PRF/5 | 0.0289 | 10.6091 |
| PSN-1 | 0.0207 | 0.2511 |
| Raji | 0.0069 | 2.0683 |
| Ramos | 0.0171 | 0.7858 |
| REC-1 | 0.0425 | 0.327 |
| RKO | 0.1066 | 2.7532 |
| RL | 0.0025 | 10.8928 |
| RMG-1 | 0.0193 | 7.9679 |
| RPMI-7951 | 0.0034 | 0.6772 |
| RPMI-8226 | 0.0137 | 2.6567 |
| RS4;11 | 0.0045 | 0.5639 |
| RT112/84 | 0.0071 | 2.2159 |
| RT4 | 0.0278 | 6.2859 |
| SCC-4 | 0.0091 | 4.9185 |
| SF268 | 0.0609 | 1.6903 |
| SH-SY5Y | 0.0774 | 0.3868 |
| SHP-77 | 0.0373 | 4.2201 |
| SiHa | 0.0419 | 8.1629 |
| SJCRH30 | 0.0056 | 6.2591 |
| SJSA-1 | 0.0124 | 7.3485 |
| SK-ES-1 | 0.0133 | 0.3715 |
| SK-MEL-2 | 0.0084 | 6.3682 |
| SK-MEL-28 | 0.0374 | 7.791 |
| SK-MEL-5 | 0.0212 | 3.6031 |
| SK-MES-1 | 0.0597 | 5.1152 |
| SK-OV-3 | 0.0077 | 6.4563 |
| SNU-1 | 0.2156 | 2.1138 |
| SNU-16 | 0.0274 | 3.7229 |
| SNU-2535 | 0.0789 | 42.6968 |
| SNU-5 | >9 | 2.7384 |
| SNU-601 | 0.0342 | 2.3784 |
| SNU-620 | 0.0163 | 25.6966 |
| SNU-81 | 0.0496 | 7.2256 |
| SNU-878 | 0.0359 | 4.2561 |
| SU-DHL-1 | 0.005 | 0.2132 |
| SU-DHL-10 | 0.0308 | 3.3368 |
| SU-DHL-2 | 0.0127 | 0.763 |
| SU-DHL-4 | 0.0059 | 1.1603 |
| SU-DHL-6 | 0.0052 | 3.4076 |
| SU-DHL-8 | 0.0172 | 1.2549 |
| SUM 159PT | 0.0014 | 3.9192 |
| SW1463 | 0.0203 | 20.3641 |
| SW1573 | 0.1354 | 18.742 |
| SW1990 | 0.0081 | 3.8759 |
| SW403 | 0.0372 | 63.8176 |
| SW-48 | 1.5621 | 38.2153 |
| SW480 | 0.0529 | 24.8823 |
| SW620 | 0.0991 | 21.9595 |
| SW626 | 0.0179 | 17.4623 |
| SW780 | 0.0145 | 10.1809 |
| SW837 | 0.0145 | 28.3994 |
| SW900 | 0.0204 | 5.333 |
| SW948 | 0.0308 | 59.0685 |
| T47D | 0.0412 | 15.2394 |
| T84 | 0.1186 | 4.0285 |
| THP-1 | 0.7708 | 1.9949 |
| Toledo | 0.013 | 0.7077 |
| TOV-21G | 0.0404 | 2.0011 |
| TT | 0.0095 | 28.2098 |
| U118 MG | 0.0038 | 5.7667 |
| U87 MG | 0.0112 | 2.6567 |
| U937 | 0.0195 | 1.4489 |
| U266B1 | 0.0303 | 9.5585 |
| UM-UC-3 | 0.0675 | 1.9364 |
| WSU-DLCL2 | 0.0035 | 0.6018 |
| YCC-10 | 0.0158 | 2.0367 |
| YCC-2 | 0.0094 | 1.3021 |
| ZR-75-1 | 0.1211 | 13.2556 |

## Claims

1. A compound of general formula (I): 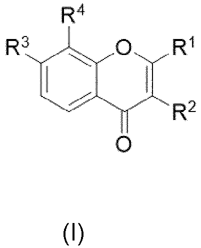 wherein
either R¹ represents A, and R² is selected from -H, -OCH₃, -OCH₂F, -OCHF₂, and -OCF₃; or
R² represents A, and R¹ is selected from -H, -OCH₃, -OCH₂F, -OCHF₂, and -OCF₃;
A represents 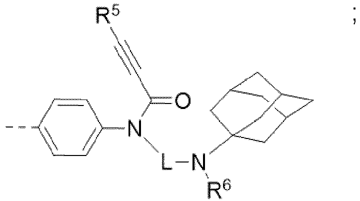
R₃ and R₄ are independently of each other selected from -O(C₁-C₆alkyl), a halogenated -O(C₁-C₆alkyl), a halogen atom and -NR⁷R⁸;
R⁵ is selected from -H, -C₁-C₆alkyl and a silyl group;
-L- represents -(CH₂)n- with n being an integer from 2 to 10;
R⁶ is selected from -C₁-C₄alkyl;
R⁷ and R⁸ are independently selected from -C₁-C₆alkyl; and
the compound is in a free form, or in a pharmaceutically acceptable salt form.

2. The compound according to claim 1, wherein the compound is of general formula (I-A) 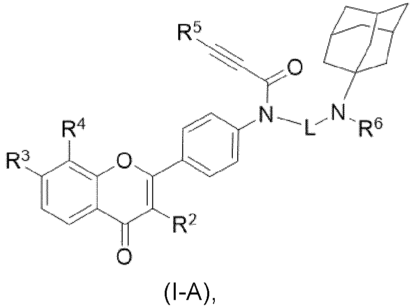 wherein R², R³, R⁴, R⁵, R⁶ and L are defined as in claim 1.

3. The compound according to claim 1 or 2, wherein R⁵ is a silyl group.

4. The compound according to any one of claims 1 to 3, wherein the silyl group is -SiR⁹R¹⁰R¹¹ with R⁹, R¹⁰ and R¹¹ being independently selected from -C₁-C₆alkyl and phenyl.

5. The compound according to any one of previous claims, wherein the silyl group is trimethylsilyl, tertbutyldimethylsilyl and triisopropylsilyl.

6. The compound according to claim 1 or 2, wherein R⁵ is -H.

7. The compound according to any one of the previous claims, wherein R₃ and R₄ are independently of each other selected from -OCH₃, -OCH₂F, -OCHF₂, -OCF₃, -F, -Cl and -NR⁷R⁸.

8. The compound according to any one of the previous claims, wherein R⁷ and R⁸ represent -CH₃.

9. The compound according to any one of the previous claims, wherein R⁶ represents -CH₃.

10. The compound according to claim 1, wherein the compound is selected from:

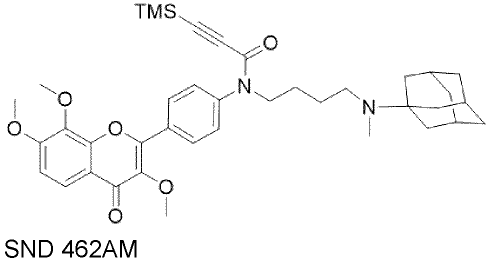

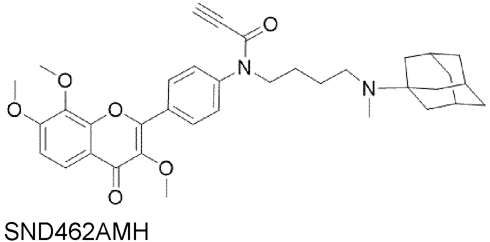

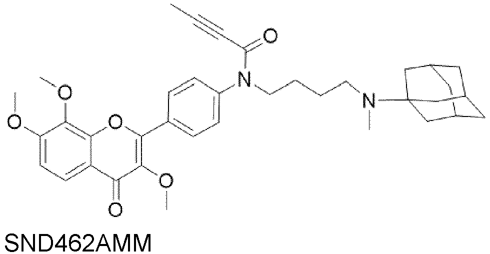

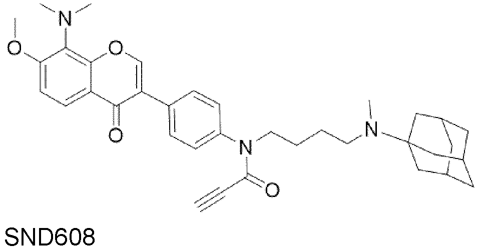

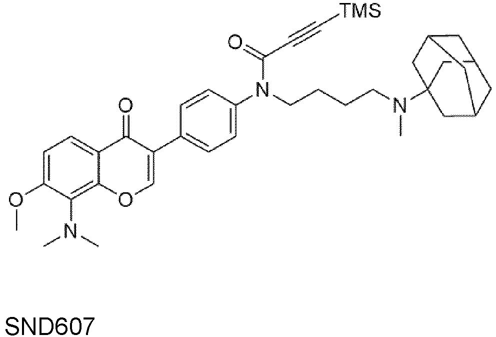

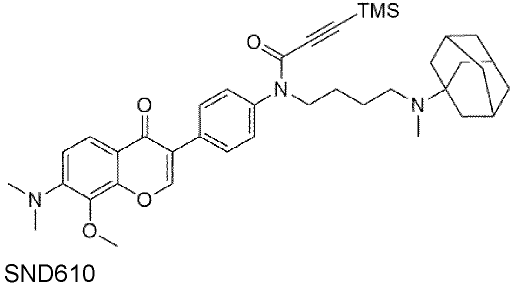

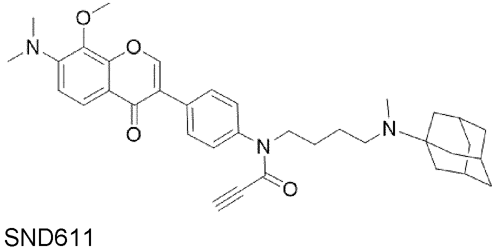

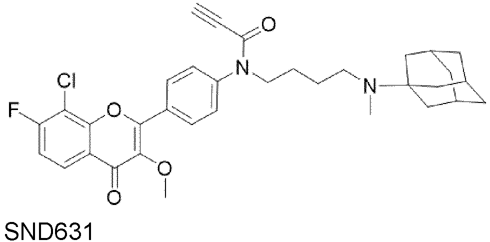

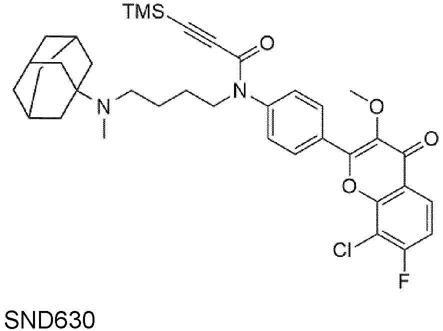

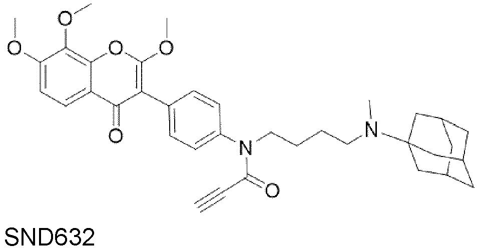

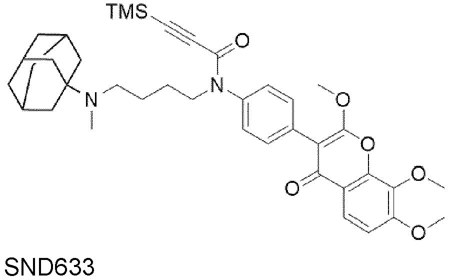

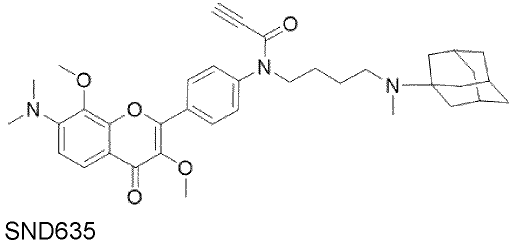

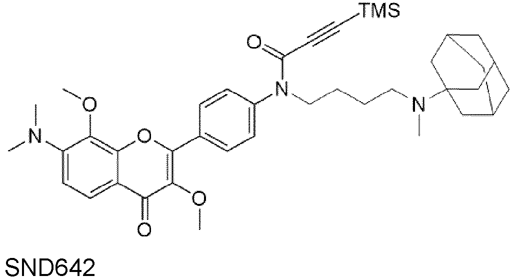

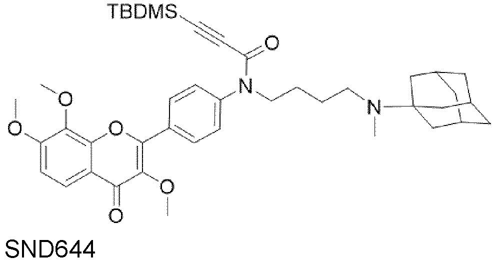

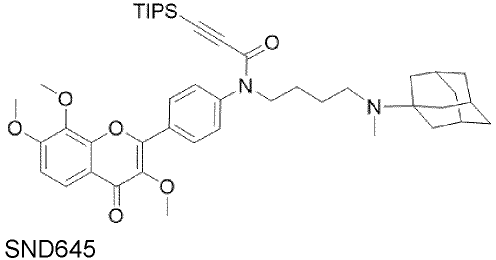

11. The compound according to any one of the previous claims, wherein the compound is in a pharmaceutically acceptable salt form, preferably selected from a hydrochloride salt form, a hydrobromide form, a mesylate salt form, an acetate salt form, and a trifluoroacetate salt form.

12. A pharmaceutical composition containing the compound according to any one of the previous claims and one or more excipients.

13. A compound according to any one of the claims 1 to 11, or a pharmaceutical composition according to claim 12 for use as a medicament.

14. A compound according to any one of the claims 1 to 11, or a pharmaceutical composition according to claim 12 for use in the treatment or prevention of cancer, including therapy-resistant cancer.

15. The compound or the pharmaceutical composition for use according to claim 14, wherein the cancer is selected from brain cancer, bladder cancer, breast cancer, hematologic cancer, head and neck cancer, intestinal cancer, kidney cancer, skin cancer, gynecologic cancer, pancreas cancer, prostate cancer, lung cancer, liver cancer, stomach cancer, soft tissue cancer, esophageal cancer, testicular cancer, bone cancer and thyroid cancer.

16. The compound or the pharmaceutical composition for use according to claim 15, wherein
• the brain cancer is selected from brain neuroblastoma, brain glioblastoma and medulloblastoma, including desmoplastic cerebellar medulloblastoma;
• the bladder cancer is bladder carcinoma,
• the breast cancer is selected from breast ductal carcinoma, including invasive breast ductal carcinoma and breast ductal carcinoma grade 3, breast pleomorphic carcinoma, breast squamous cell carcinoma, and metastatic adenocarcinoma,
• the hematologic cancer is selected from
∘ blood leukemia, including acute myeloid leukemia (AML), chronic myeloid leukemia (CML), acute lymphoblastic leukemia (ALL) including B-cell and T-cell, and chronic eosinophilic leukemia,
∘ blood lymphoma including Hodgkin lymphoma and non- Hodgkin lymphoma, in particular non-Hodgkin lymphoma, including B-cell lymphoma such as, Burkitt lymphoma, diffuse large B-cell lymphoma, and mantel cell lymphoma, and T-cell lymphoma such as, cutaneous T-cell lymphoma, and anaplastic large cell lymphoma, and
∘ blood myeloma in particular plasmacytoma and multiple myeloma,
• the head and neck cancer is selected from tongue squamous cell carcinoma and pharynx squamous cell carcinoma,
• the intestinal cancer is selected from duodenal cancer, in particular duodenal adenocarcinoma, colorectal cancer in particular colorectal adenocarcinoma including Duke's type D and Duke's type C, colon carcinoma including colon adenocarcinoma, rectal carcinoma in particular rectal adenocarcinoma, and cecum carcinoma in particular cecum adenocarcinoma,
• the pancreatic cancer is pancreatic adenocarcinoma (ductal adenocarcinoma),
• the liver cancer is hepatocarcinoma,
• the skin cancer is skin melanoma;
• the lung cancer is selected from small cell lung carcinoma, carcinoid, pleural mesothelioma, and non-small cell lung carcinoma, in particular adenocarcinoma, large cell carcinoma, squamous cell carcinoma and bronchoalveolar carcinoma,
• the gynecologic cancer is selected from
∘ cervical cancer,
∘ uterine/endometrial cancer, in particular endometrial adenocarcinoma and uterine choriocarcinoma, and
∘ ovarian cancer in particular ovarian teratocarcinoma and ovarian adenocarcinoma including ovarian clear-cell adenocarcinoma, ovarian serous cyst adenocarcinoma including high grade ovarian serous adenocarcinoma, ovarian endometrioid adenocarcinoma, ovarian primary malignant adenocarcinoma, and ovarian serous cystadenocarcinoma,
• the stomach cancer is selected from stomach carcinoma, in particular adenosquamous carcinoma and cholangiocellular carcinoma, and stomach adenocarcinoma including signet ring cell stomach adenocarcinoma,
• the soft tissue cancer is selected from fibrosarcoma, rhabdomyosarcoma including embryonal rhabdomyosarcoma, liposarcoma, biphasic synovial sarcoma, epithelioid sarcoma and Ewig sarcoma,
• the esophageal cancer is esophageal squamous cell carcinoma,
• the testicular cancer is testicular embryonal carcinoma,
• the bone cancer is selected from osteosarcoma and Ewing sarcoma, and
• the thyroid cancer is selected from hereditary thyroid gland medullary carcinoma and anaplastic carcinoma.
